# EUROPEAN PATENT SPECIFICATION

(11) **EP 2 701 516 B1**
(45) Date of publication and mention of the grant of the patent: **13.12.2017**
(21) Application number: 12777000.6
(22) Date of filing: 25.04.2012
(51) Int. Cl.: A61L 27/38, A61K 45/06, A61K 35/17, A61K 35/28, A61P 19/00, A61K 31/616, A61L 27/54

(54) **COMPOSITIONS FOR IMPROVED TISSUE REGENERATION BY SUPPRESSION OF INTERFERON -GAMMA AND TUMOR NECROSIS FACTOR-ALPHA**
ZUSAMMENSETZUNGEN FÜR VERBESSERTE GEWEBEREGENERATION DURCH UNTERDRÜCKUNG DES GAMMA-INTERFERONS UND DES ALPHA-TUMORNEKROSEFAKTORS
COMPOSITIONS POUR LA RÉGÉNÉRATION TISSULAIRE PAR SUPPRESSION DE L'INTERFÉRON-GAMMA ET DU FACTEUR DE NÉCROSE TUMORALE ALPHA

(30) Priority: 25.04.2011 US 201161478894 P
(43) Date of publication of application: 05.03.2014
(73) Proprietor: University Of Southern California, Los Angeles, California 90015 (US)
(72) Inventor: SHI, Songtao, Thousand Oaks CA 91362 (US); LIU,Yi, Alhambra CA 91803 (US); WANG, Lei, Monterey Park CA 91755 (US)
(74) Representative: Viering, Jentschura & Partner mbB Patent- und Rechtsanwälte
(86) International application number: PCT/US2012/035036
(87) International publication number: WO 2012/149046

(56) References cited:
- US-A1- 2005 186 207
- US-A1- 2008 124 374
- US-A1- 2009 069 903
- US-A1- 2009 130 067
- TAKAYOSHI YAMAZA ET AL: "Pharmacologic Stem Cell Based Intervention as a New Approach to Osteoporosis Treatment in Rodents", PLOS ONE, vol. 3, no. 7, 9 July 2008 (2008-07-09), page e2615, XP055178922, DOI: 10.1371/journal.pone.0002615
- YI LIU ET AL: "Mesenchymal stem cell-based tissue regeneration is governed by recipient T lymphocytes via IFN-[gamma] and TNF-[alpha]", NATURE MEDICINE, vol. 17, no. 12, 20 November 2011 (2011-11-20), pages 1594-1601, XP055178910, ISSN: 1078-8956, DOI: 10.1038/nm.2542

## Description

### CROSS-REFERENCE TO RELATED APPLICATIONS

This application claims an invention which was disclosed in Provisional Application Number 61/478,894 filed April 25, 2011.

### FIELD OF THE INVENTION

The invention pertains to the field of regenerative medicine.

### BACKGROUND OF THE INVENTION

The field of regenerative medicine seeks to repair, replace, or regenerate tissues and organs damaged by injuries or diseases. To this end, cell-based regenerative medicine has been recognized as a promising approach to meet the many challenges of tissue regeneration. Within cell-based regenerative medicine, stem cells have emerged as a promising source of cells. To date, culture-expanded osteoprogenitor cells have been used in conjunction with scaffolds for tissue engineering with some success (26-28). How, the main challenge of cell-based tissue regeneration is to form large quantities of high-quality tissue structures that match the body's functional needs (29, 30). Current methodologies have yet to overcome the challenges of the field.

In view of the above, there is still a substantial need for improved compositions and methods for tissue regeneration, especially methods and compositions which permit the formation of significant quantities of high quality tissue structures that meet the body's functional needs.

In addition, there is also a need to further clarify the role of immune responses in cell-based tissue regeneration. The invention is defined by the claims.

### BRIEF DESCRIPTION OF THE DRAWINGS

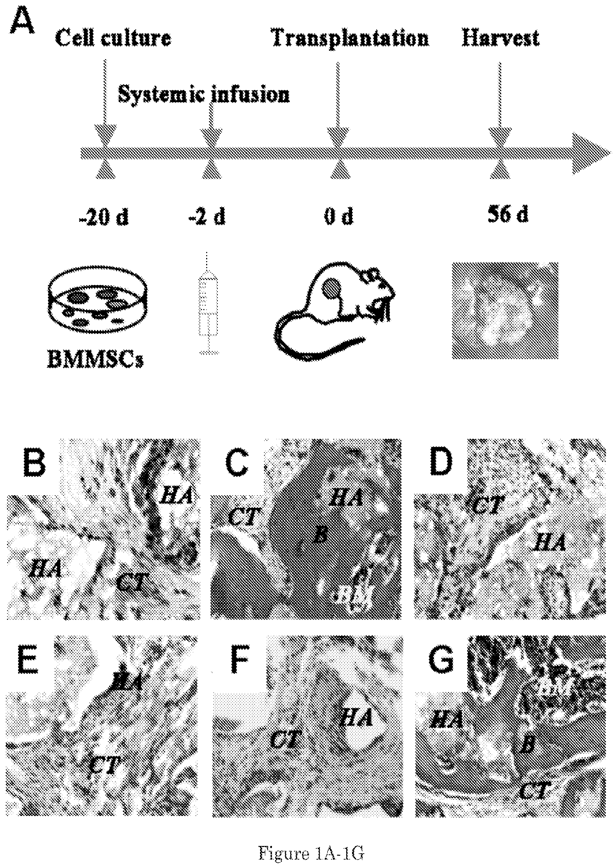

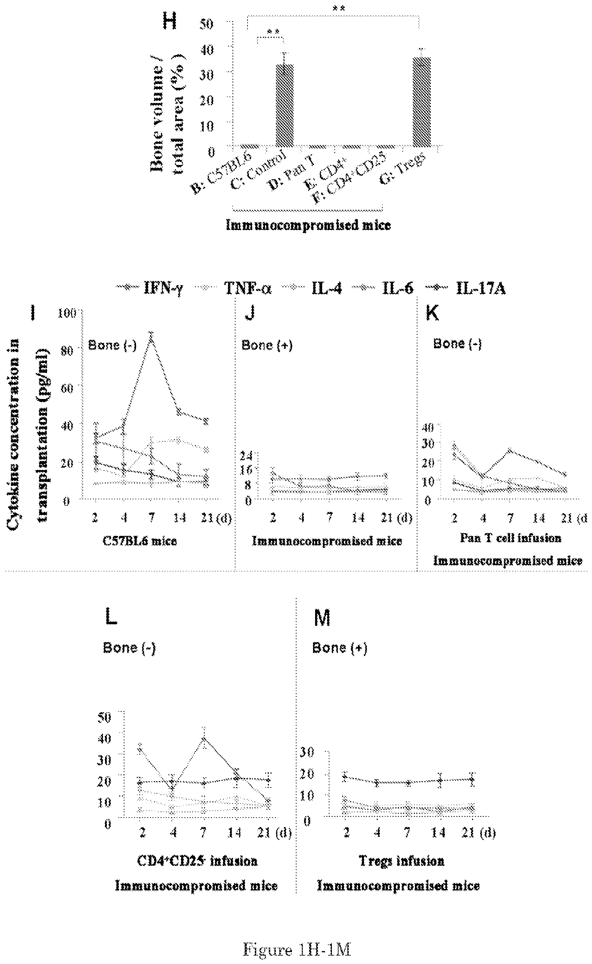

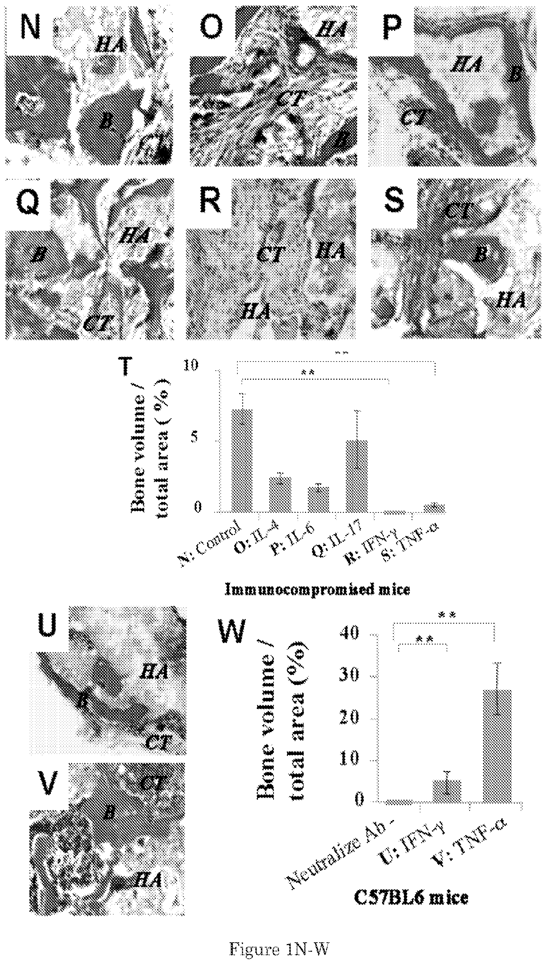

**Figure 1** shows that T cells regulated BMMSC (bone marrow mesenchymal stem cell) -mediated bone formation *in vivo.* (A) Scheme of assessing BMMSC-based tissue regeneration. (B) When BMMSCs (4×10⁶ cells) from the littermate were subcutaneously implanted into C57BL6 mice using hydroxyapaptite/tricalcium phosphate (HA/TCP, 40mg/each implant) as a carrier, they were not able to regenerate bone at 8 weeks post implantation. H&E staining showed connective tissue surround HA/TCP particle (*HA*) in the BMMSC implants. (C) Under the same implantation condition, BMMSCs formed substantial amount of bone (B), bone marrow (*BM*), and connective tissue (*CT*) around HA/TCP *(HA)* in immunocompromised mice at 8 weeks post implantation. (D-F) Infusion of Pan T (D), CD4⁺ (E), and CD4⁺CD25⁻ (F) cells (1×10⁶) *via* tail vein at 2 days prior to BMMSC implantation blocked BMMSC-mediated bone formation in immunocompromised mice. H&E staining showed connective tissue (*CT*) surround HA/TCP particle (*HA*) in the BMMSC implants. (G) Infusion of CD4⁺CD25⁺Foxp3⁺ regulatory T cells (Tregs, 1×10⁶) *via* tail vein at 2 days prior to BMMSC implantation showed no inhibitive effect on bone (B) and bone marrow (*BM*) formation in immunocompromised mice. (H) Image J semi-quantitative analysis indicated marked bone formation in BMMSC implants in immunocompromised mice (C: Control) and Treg-infused immunocompromised mice (G: Tregs). However, there was no bone formation in BMMSC implants in C57BL6 mice (B: C57BL6), Pan T cell-infused immunocompromised mice (D: Pan T), CD4⁺ cell-infused immunocompromised mice (E: CD4⁺), and CD4⁺CD25⁻ cell-infused immunocompromised mice (F: CD4⁺CD25⁻). (I) When BMMSCs were subcutaneously implanted into C57BL6 mice, there were sharply elevated expression of IFN-γ and increased expression of TNF-α in the BMMSC implants from 4 to 14 days post implantation as evidenced by ELISA analysis. However, there were no significant changes in the levels of IL-4, IL-6, and IL-17A in the BMMSC implants. (J-M) When BMMSCs were subcutaneously implanted into immunocompromised mice, there was no significant change in levels of IFN-γ, TNF-α, IL-4, IL-6, and IL-17A (J). With infusion of Pan T cells or CD4⁺CD25⁻ cells (1×10⁶) into immunocompromised mice, the levels of IFN-γ and TNF-α were increased in BMMSC implants, along with no significant change for the levels of IL-4, IL-6, and IL-17A (K, L). Infusion of Tregs appeared no effect on the levels of IFN-γ, TNF-α, IL-4, IL-6, and IL-17A in the BMMSC implants in immunocompromised mice (M). (N-T) IFN-γ, TNF-α, IL-4, IL-6, and IL-17A (200 ng) were mixed with 250 µl hydrogel containing BMMSCs (4×10⁶) and were subsequently implanted into immuncompromised mice using HA/TCP as a carrier. As expected that BMMSC/HA/TCP positive control group showed marked bone formation (*B*) around HA/TCP (*HA*) at 8 weeks post implantation (N). IL-4 and IL-6 treated BMMSCs showed a reduction in new bone formation (O, P) and IL-17 treatment appeared no inhibitive effect on BMMSC-mediated bone formation (Q). Moreover, IFN-γ treatment resulted in a completely blockage of new bone formation (R) and TNF-α treatment led to a very limited bone formation in BMMSC implants (S). Image J semi-quantitative analysis showed the amount of new bone formation in each group (T). (U-W) When IFN-γ and TNF-α neutralizing antibodies (300 µg/mouse) were injected into C57BL6 mice for three times *via* I.P. starting at 2 days prior to BMMSC implantation, new bone formation was observed in BMMSC implants at 8 weeks post implantation (U, V). The amount of new bone formation was shown by semi-quantitative analysis of image J analysis (W). (***P*<0.01, N=5).

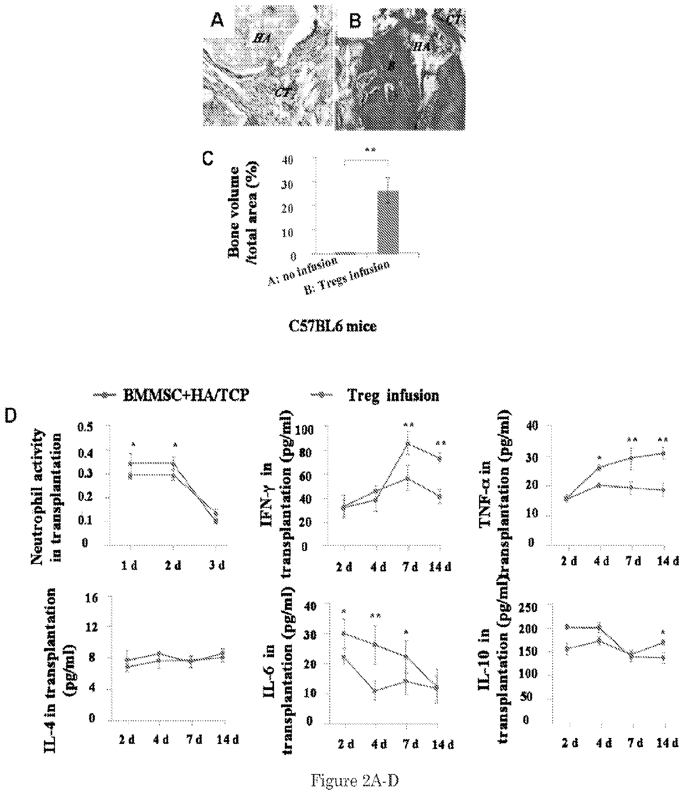

**Figure 2** shows that Regulatory T cell (Treg) improved BMMSC-mediated bone formation in C57BL6 mice. (A-C) Subcutaneous BMMSC implantation in C57BL6 mice failed to generate any bone (A), and only connective tissue (*CT*) around HA/TCP (*HA*) were observed. However, Infusion of CD4⁺CD25⁺Foxp3⁺ regulatory T cells (1×10⁶) *via* the tail veins at 2 days prior to the BMMSC/HA/TCP implantation resulted in a marked new bone formation (*B*) around HA/TCP particles *(HA)* and connective tissue (*CT*) in BMMSC implants at 8 weeks post implantation (B). Image J semi-quantitative analysis indicated relative amount of bone formation in BMMSC implants (C). (D) ELISA analysis showed that Treg infusion reduced levels of IFN-γ and TNF-α in the BMMSC implants at 7-14 days post implantation when compared to un-treated control group. It appeared the levels of IL-4 and IL-10 had no significant changes among these groups. In addition, Treg infusion reduced the number of neutrophil cells and levels of IL-6 in the BMMSC implants at 1-2 and 4-7 days post implantation, respectively. (**P*<0.05, ***P*<0.01, N=5).

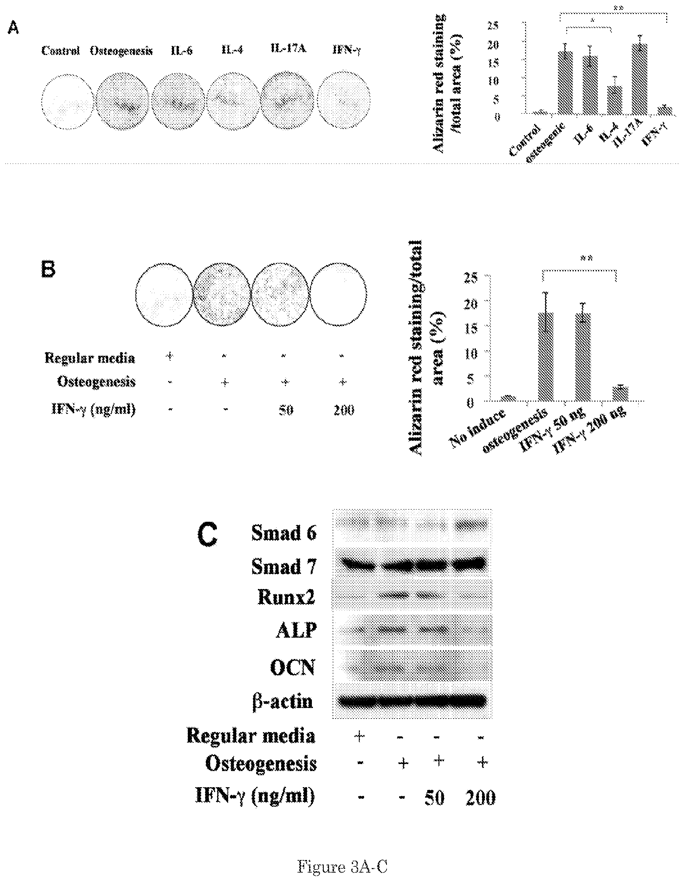

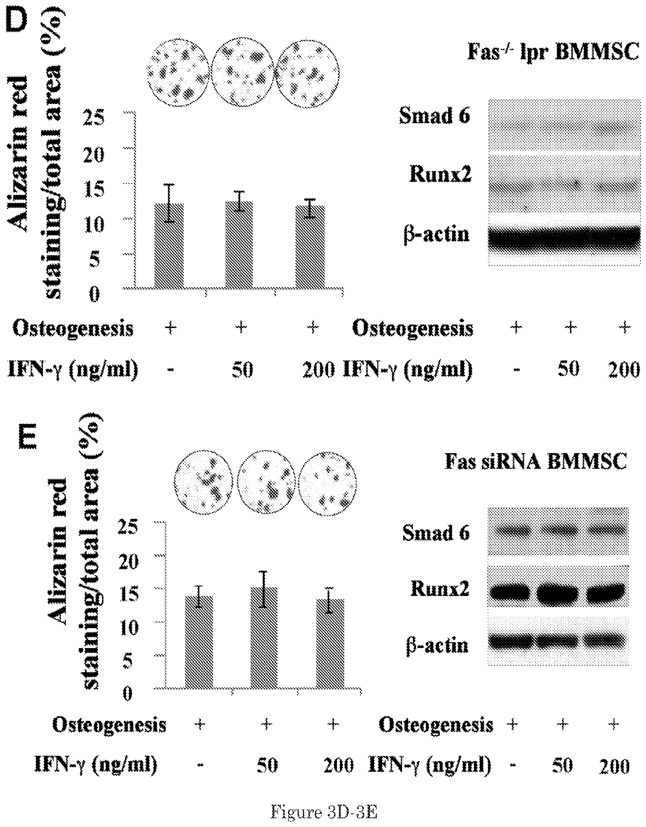

**Figure 3** shows that IFN-γ inhibited osteogenic differentiation of BMMSCs. (A) When BMMSCs were co-cultured with 200 ng/ml IL-4, IL-6, IL-17A, and IFN-γ in osteogenic media for 14 days, alizarin red staining showed that IFN-γ treatment significantly inhibited mineralized nodule formation when compared to osteogenic inductive (osteogenesis), IL-4, IL-6, and IL-17A groups. It appeared that IL-4 treatment reduced mineralized nodule formation. But IL-6 and IL-17A treatment had no inhibition effect on the mineralized nodule formation. (B) When 50 ng/ml IFN-γ was used to treat BMMSCs, there was no significant inhibitive effect on mineralized nodule formation compared to osteogenic inductive group as evidenced by alizarin red staining. However, 200 ng/ml IFN-γ treatment showed a significant reduction in mineralized nodule formation. (C) Western blot analysis showed that IFN-γ at 200 ng/ml elevated Smad 6 expression and inhibited expression levels of osteogenic genes Runx2, ALP and OCN, which were not observed in 50 ng/ml IFN-γ treatment group. Expression level of Smad 7 was not altered in IFN-γ-treated BMMSCs. (D, E) IFN-γ treatment failed to block osteogenesis and alter expression levels of Smad 6 and Runx2 in Fas^{-/-} BMMSCs derived from *lpr* mice (D) and Fas knock down BMMSCs by siRNA (E) as assessed by alizarin red staining and Western blot analysis, respectively. (**P*<0.05, ***P*<0.01, N=5).

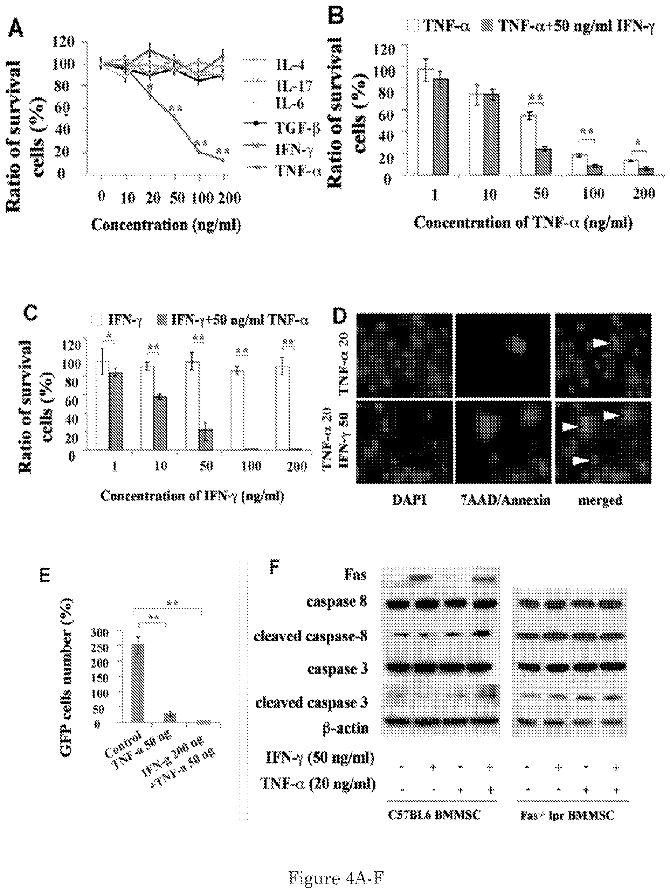

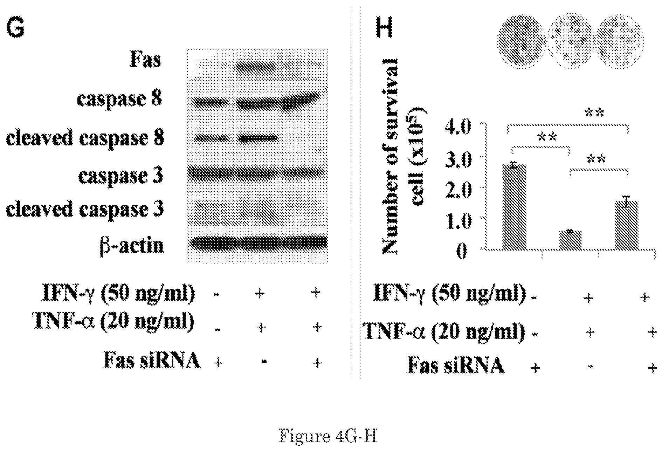

**Figure 4** shows that IFN-γ synergistically enhanced TNF-α-induced BMMSC apoptosis *via* Fas apoptotic pathway. (A) IL-4, IL-6, IL-17A, TGF-β1, IFN-γ, and TNF-α at various concentrations (1, 10, 20, 50, 100, 200 ng/ml) were cultured with BMMSCs for three days. BMMSC viability was assessed by toluidine blue staining. Only TNF-α induced a dose-dependent cell death and other cytokines showed no effect on BMMSC death. (B) TNF-α (50 ng/ml to 200 ng/ml) induced BMMSC apoptosis was synergistically enhanced by IFN-γ treatment (50 ng/ml) as assessed by toluidine blue staining. (C) IFN-γ (1-200 ng/ml) was not able to induce BMMSC apoptosis. With adding TNF-α, (50 ng/ml), a dose dependent BMMSC death was observed from 1 to 50 ng/ml IFN-γ groups. When IFN-γ concentration reached higher than 100 ng/ml, there was no any BMMSC survived in the cultures. (D) Representative images showed that IFN-γ (50 ng/ml) accelerated TNF-α (20 ng/ml) induced BMMSC apoptosis (triangles, TNF-α 20 ng/ml /IFN-γ 50 ng/ml) as evidenced by 7AAD/Annexin staining when compared to TNF-α treatment group (TNF-α 20 ng/ml). (E) When BMMSCs (4×10⁶) were subcutaneously implanted into immunocompromised mice with matrigel containing TNF-α (50 ng/ml) showed a marked reduction in the numbers of survived BMMSCs compared to untreated control group at 30 days post implantation. Moreover, combined treatment of TNF-α (50 ng/ml) and IFN-γ (200 ng/ml) resulted in a completely no BMMSC survived at 30 days post implantation. (F) The synergy role of IFN-γ in TNF-α-induced apoptosis was through Fas pathway. IFN-γ (50 ng/ml) treatment induced up-regulated expression of Fas in wild type BMMSCs along with no activation of caspase 3 and caspase 8. TNF-α (20 ng/ml) treatment showed slightly increase in Fas expression and caspase 3 activity as evidenced by Western blot analysis. When co-stimulated with IFN-γ and TNF-α, expression levels of Fas, cleaved caspase 8 and 3 were significantly elevated when compared to TNF-α treatment group. Interestingly, BMMSCs derived from Fas^{-/-} lpr mice showed no response to IFN-γ and TNF-α, treatment in terms of expression of cleaved caspase 8 and 3. (G, H) When Fas expression was partially blocked by siRNA, Western blot analysis showed that expression levels of cleaved caspase 8 and 3 were reduced in IFN-γ/TNF-α-treated group (G), along with a significantly reduced cell death as assessed by toluidine blue staining (H). (**P*<0.05, ***P*<0.01, N=5).

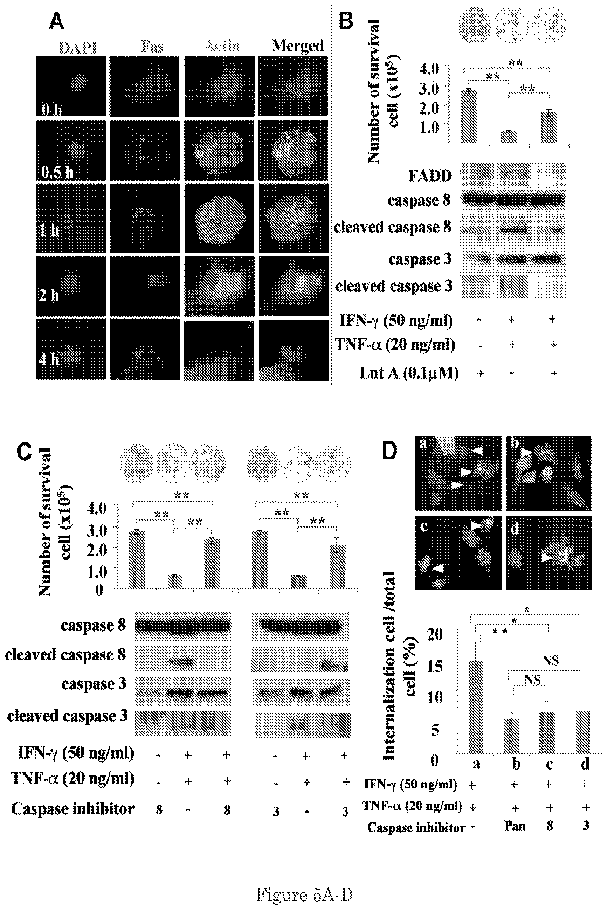

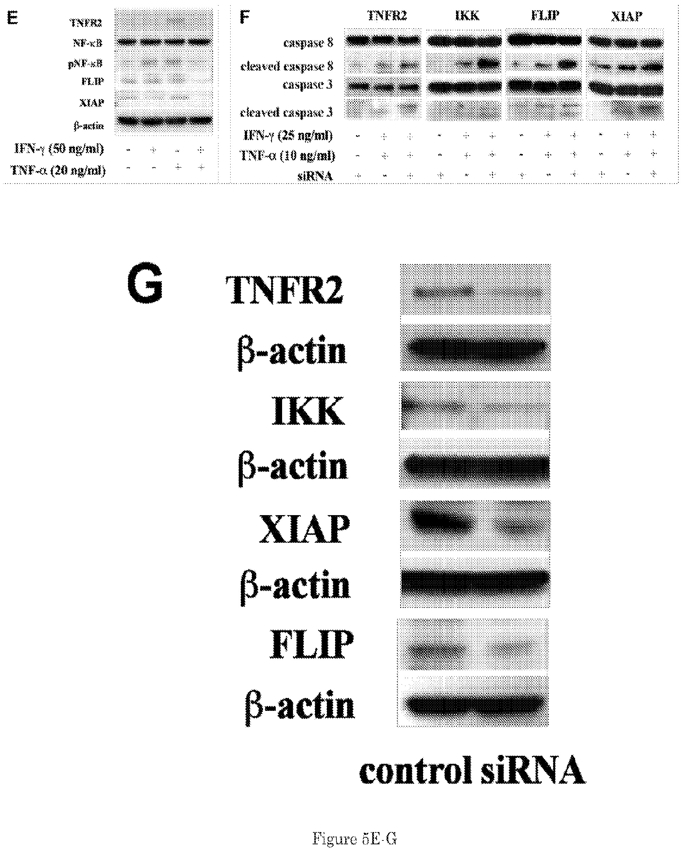

**Figure 5** shows that IFN-γ synergistically enhanced TNF-α-induced BMMSC apoptosis *via* inhibition of TNFR2/NFκB pathway and Fas internalization. (A) Immunocytochemical staining showed that Fas and actin were localized on BMMSC surface and in cytoplasm, respectively (0 h). IFN-γ (50 ng/ml) and TNF-α (20 ng/ml) treatment resulted in Fas internalization and clustering in BMMSCs as shown in indicated time points (0.5-4 h). A marked BMMSC apoptosis was observed at 2- 4 h IFN-γ/TNF-α treatment. (B) When endocytosis inhibitor Lnt A was used to treat BMMSC for 3 hours, IFN-γ/TNF-α-induced cell death and activation of cleaved caspase 8 and 3 were significantly reduced as assessed by toluidine blue staining and Western blot analysis. (C) Caspase 8 inhibitor can reduce levels of cleaved caspase 8 and 3 in IFN-γ/TNF-α-treated BMMSCs, which was associated with rescuing IFN-γα/TNF-α-induced cell death as shown by toluidine blue staining. Caspase 3 inhibitor was also capable of rescuing IFN-γ/TNF-α-induced BMMSC death with partially inhibition of cleaved caspase 3 and no inhibitive effect on cleaved caspase 8. (D) Blockage of caspase activities using pan caspase inhibitor (b), caspase 8 inhibitor (c), or caspase 3 inhibitor (d) reduced number of Fas internalization when compared to control group (a). Immunocytochemical staining was used to identify number of BMMSCs with Fas internalization. (E) Western blot analysis showed that IFN-γ/TNF-α treatment resulted in a decreased expression of TNFR2, phosphorylated NFκB (pNFκB), FLIP, and XIAP when compare to TNF-α-treated BMMSCs. (F) Inhibition of TNFR2, IKK, FLIP, and XIAP using siRNA led to up-regulation of cleaved caspase 8 and 3 in IFN-γ (25 ng/ml)/TNF-α (10 ng/ml) treated BMMSCs. (G) Western blot analysis confirmed efficacy to siRNA in inhibiting TNFR2, IKK, FLIP, and XIAP. (**P*<0.05, ***P*<0.01, N=5).

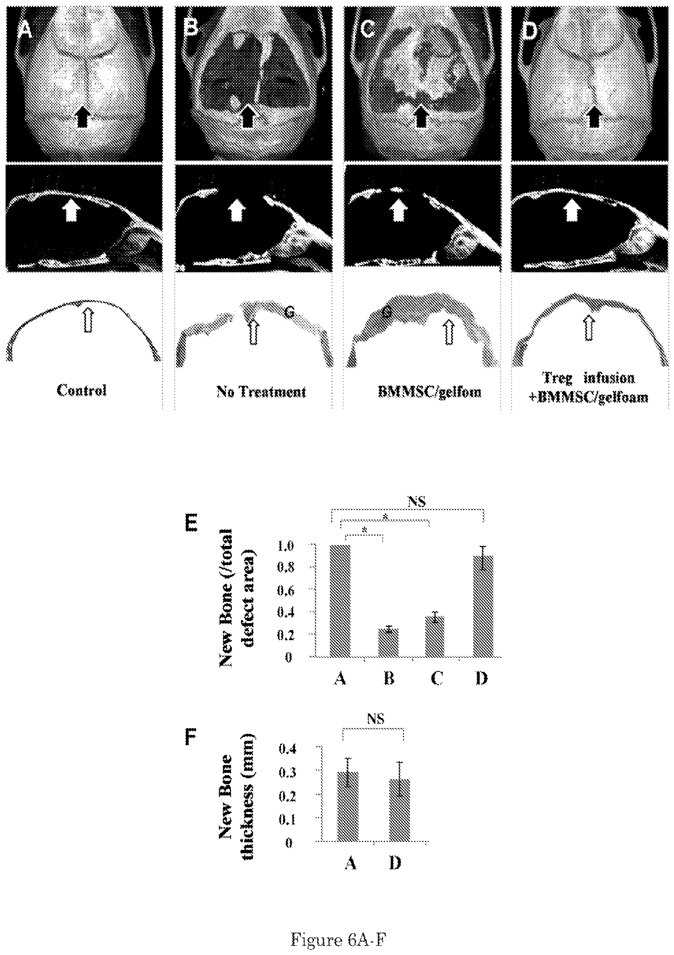

**Figure 6** shows that Treg infusion improved BMMSC-mediated bone formation for repairing calvarial bone defects in C57BL6 mice. (A) Calvarial bones and suture images of coronal microCT image (upper panel), Sagittal microCT (middle panel) and coronal H&E staining (lower panel). (B) 7×8 mm critical-sized calvarial bone defects were generated in un-treated control mice, and very limited amount of bone formation was identified at 12 weeks post-surgery (arrow). (C) With implantation of 4×10⁶ BMMSCs using gelfoam as a carrier, there was certain amount bone regeneration at 12 weeks post implantation, but fail to completely repair the calvarial defects (arrow). (D) 1×10⁶ Tregs were infused into the recipient mice via tail vein at 2 days prior to BMMSC (4×10⁶)/gelfoam implantation, a totally repairing of the calvarial defects was observed at 12 weeks post implantation (arrow). (E) MicroQCT quantitative analysis showed amount of bone formation in each group. (F) Image J semi-quantitative analysis indicated that BMMSC/gelfoam implantation with systemic Treg infusion group had similar bone thickness as normal calvarial bone. (**P*<0.01, N=5; *G*: galform; *B*: bone).

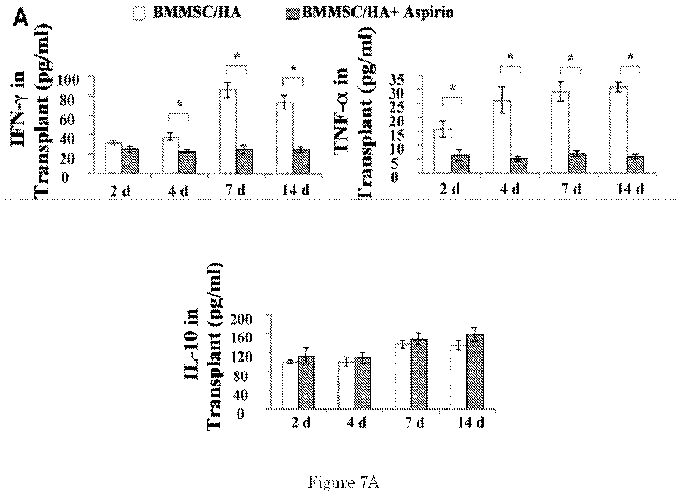

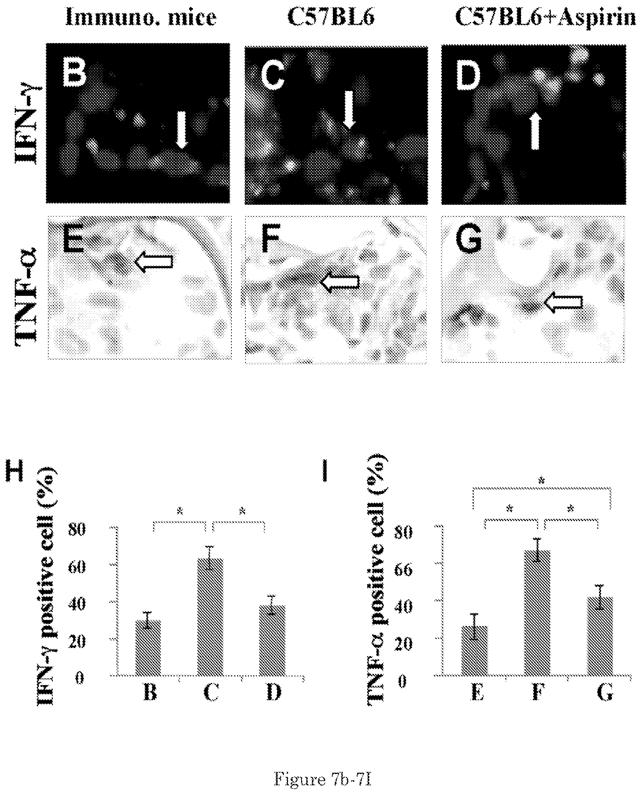

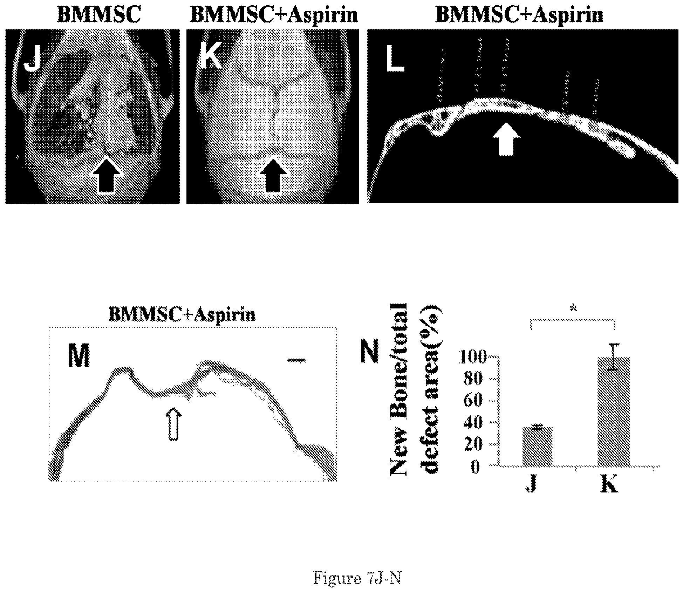

**Figure 7** shows that aspirin treatment significantly improved BMMSC-based bone regeneration via inhibition of IFN-γ and TNF-α. (A) ELISA analysis showed that aspirin reduced levels of IFN-γ and TNF-α, but not for IL-10, in the BMMSC implants when compared to un-treated control group from 2 to 14 days post implantation. (B-I) Immunohistochemical staining showed that the numbers of IFN-γ and TNF-α positive cells were higher in BMMSC implants in C57BL6 mice in comparison to BMMSC implants in immunocompromised mice. There were significantly decreased levels of IFN-γ and TNF-α in aspirin treated group when compare to untreated control group in C57BL6 mice. (J-N) 4×10⁶ BMMSCs were cultured with 50 µg/ml aspirin for 2 days and subsequently seeded into 7×8 mm calvarial bone defect areas using gelfoam as carrier, which contained 100 µg aspirin. At 12 weeks post implantation, microQCT analysis showed that calvarial defects were completely repaired (K). However, BMMSC implantation group exhibited a partial repairing of calvarial bone defects (J). Sagittal microQCT image and coronal H&E staining showed repaired bone structure (L, M). Image J semi-quantitative analysis showed relative amount of bone formation in calvarial defect area (N). (^{*}*P*<0.01, N=5).

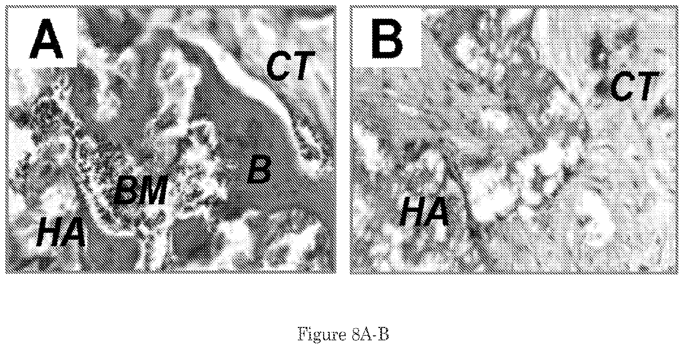

**Figure 8** shows Autologous BMMSCs failed to regenerate bone in C57BL6 mice. (A) When BMMSCs were subcutaneously implanted into immunocompromised mice using hydroxyapatite tricalcium phosphate (HA/TCP) as a carrier, bone (*B*) and bone marrow (*BM*) were regenerated around HA/TCP (*HA*). (B) When autologous BMMSCs were implanted into C57BL6 subcutaneously, there was no bone formation. Only connective tissue (*CT*) was found around HA/TCP (*HA*).

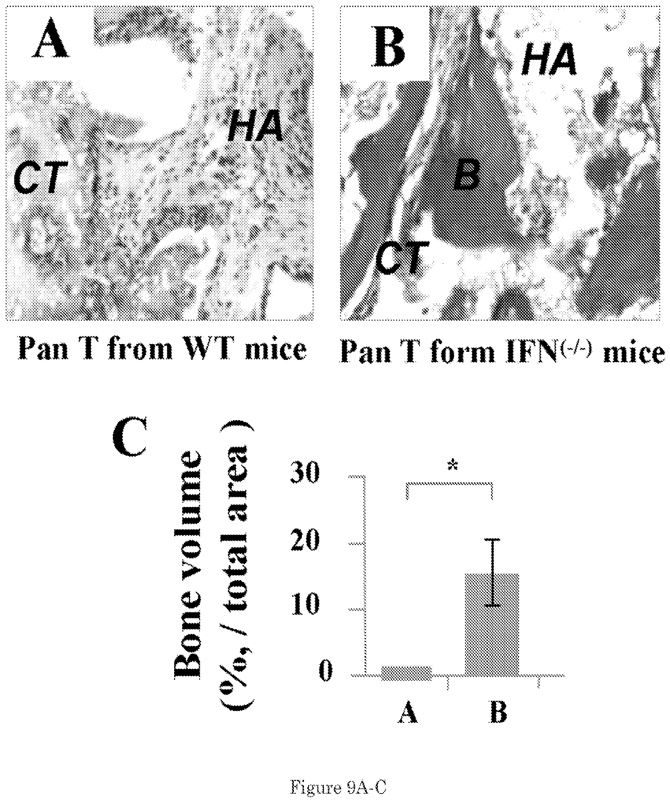

**Figure 9** shows that IFN-γ deficient T cells could not block BMMSC-mediated bone formation in immunocompromised mice. (A) Infusion of Pan T cells (1×10⁶) to immunocompromised mice 2 days prior to BMMSC implantation (2×10⁶ with 40 mg HA/TCP), there was no bone formation. H&E staining showed connective tissue (*CT*) around HA/TCP (*HA*). (B) Infusion of IFN-γ deficient Pan T cells derived from IFN-γ null mice was not able to block BMMSC-mediated bone formation. H&E staining showed that newly formed bone (*B*) around HA/TCP (*HA*). (C) Image J semi-quantitative analysis showed the amount of bone formation. (**P*<0.01, N=5).

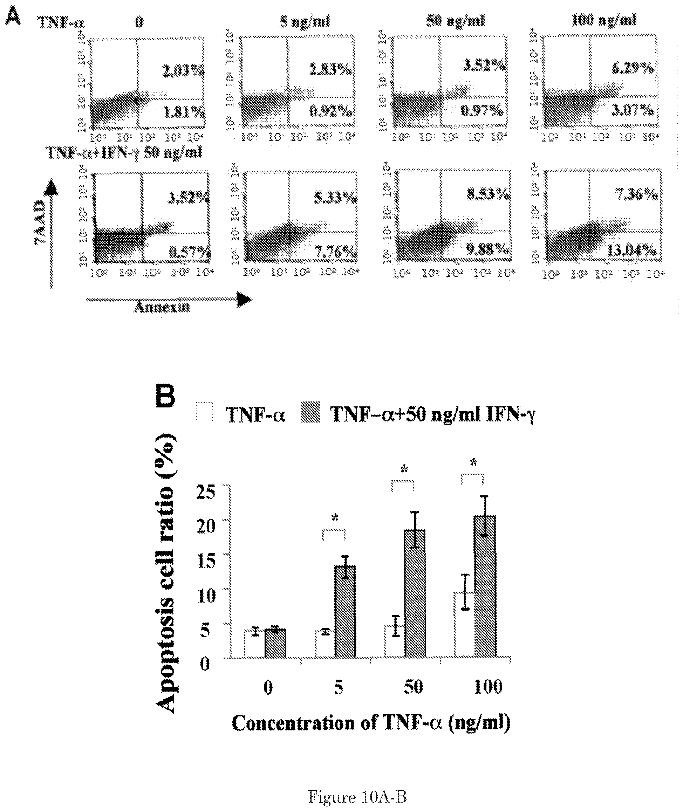

**Figure 10** shows IFN-γ synergistically enhanced TNF-α-induced BMMSC apoptosis *in vitro.* (A) The Flow cytometric analysis confirmed that the TNF-α at 0, 5, 50, 100 ng/ml caused dose-dependent BMMSC apoptosis. Additional IFN-γ (50 ng/ml) could synergize TNF-α-induced apoptosis. (B) Statistic analysis showed that 50 ng/ml IFN-γ significantly enhanced TNF-α-induced BMMSC apoptosis. (**P*<0.01, N=4).

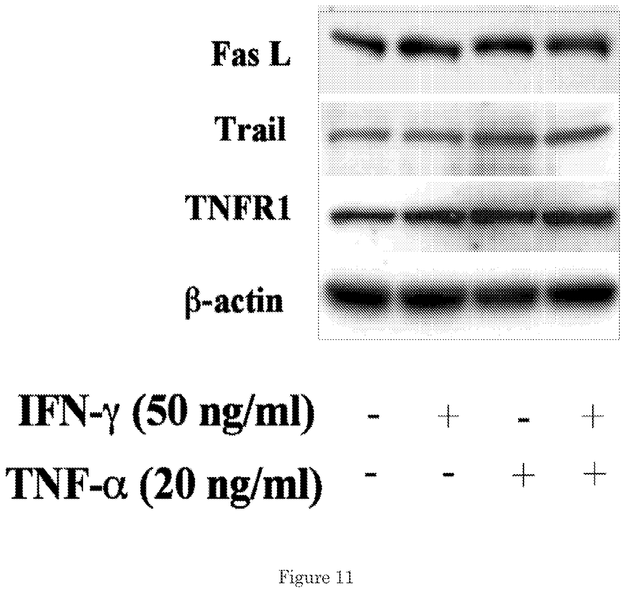

**Figure 11** shows that expression levels of Fas L, Trail, and TNFR1 in IFN-γ/TNF-α-treated BMMSCs. Western blot analysis showed that IFN-γ/TNF-α treatment did not affect expression levels of Fas L, TNFR21 and Trail.

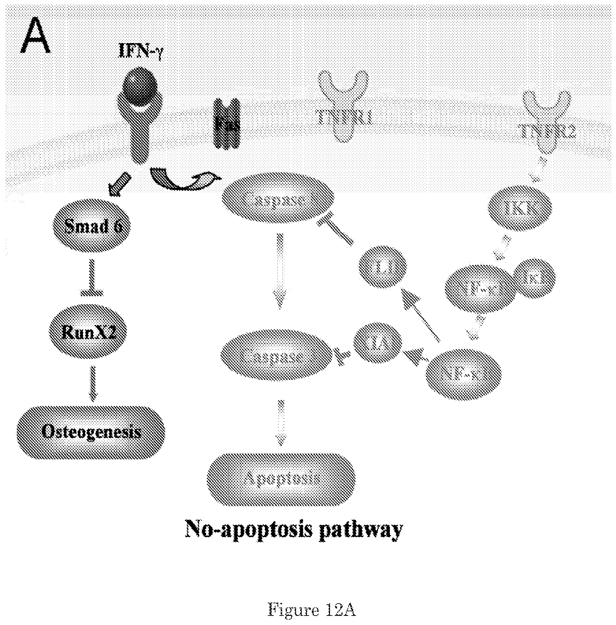

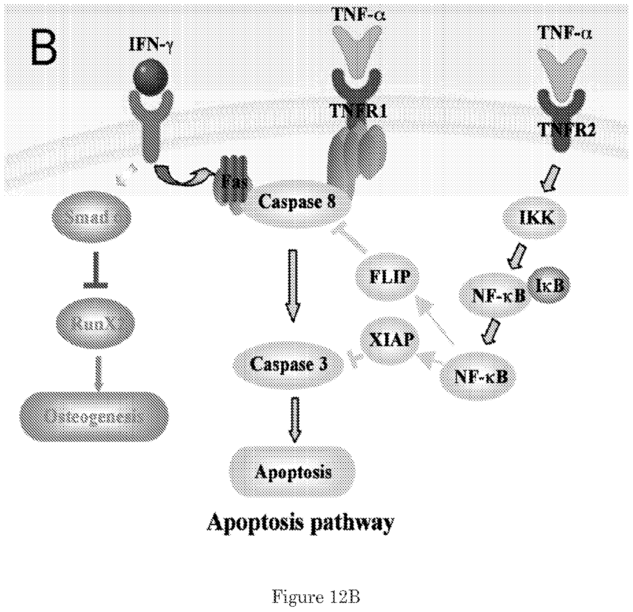

**Figure 12** shows a schematic diagram of IFN-γ induced non apoptotic pathway (A) and IFN-γ/TNF-α induced CD95 apoptotic pathway (B).

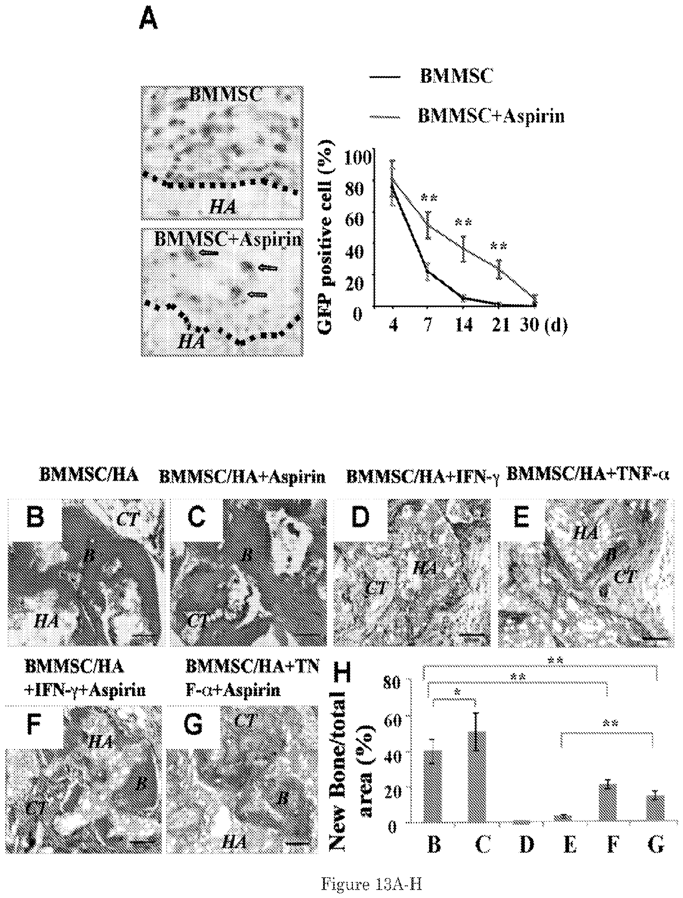

**Figure 13** shows that aspirin treatment improved BMMSC survival and bone regeneration *in vivo.* (A) When GFP positive BMMSCs were subcutaneously implanted for 30 days, the aspirin-treated group (50 µg/ml aspirin pre-treated for 2 days, 100 µg aspirin co-implanted) showed significantly increased numbers of survival cells compared to untreated control group from 4 to 30 days post implantation. (B-G) BMMSCs were treated with 50 µg/ml aspirin for 2 days and then combined with HA/TCP particles to be implanted into immunocompromised mice subcutaneously. The amount of new bone formation was increased in aspirin-treated group compare to un-treated control group (B, C). Co-implantation of 200 ng IFN-γ and TNF-α with BMMSCs inhibited subcutaneous bone formation in immunocompromised mice (D, E), however, pre-treatment BMMSCs with 50 µg/ml aspirin and co-implantation of 100 µg aspirin resulted in a significant rescuing of BMMSC-mediated bone formation (F, G). Image J semi-quantitative analysis showed relative amount of bone formation (H). (**P*<0.05, ***P*<0,01, N=5).

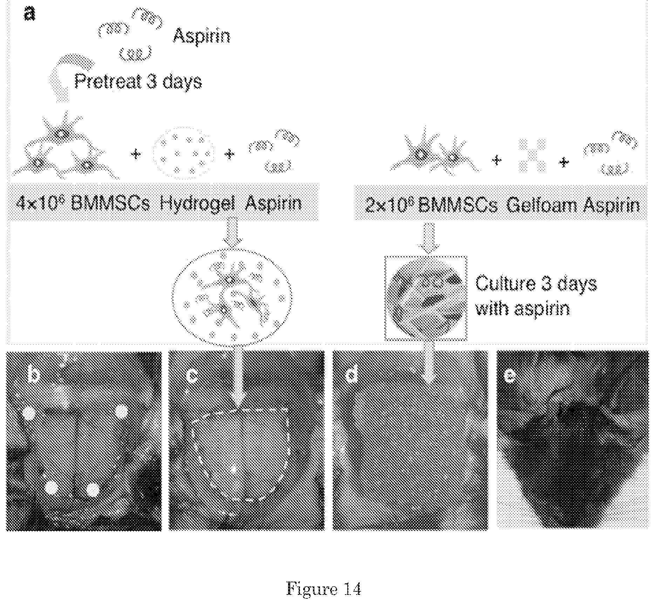

**Figure 14** shows schematic diagram of BMMSC/aspirin implantation to repair calvarial defects. (a) BMMSC/hydrogel/aspirin and BMMSC/gelfoam/aspirin were generated for implantation. (b) Calvarial defects were generated along the yellow dots in C57BL6 mice. (c) Implantation of BMMSC/hydrogel/aspirin complex to calvarial bone defects in C57BL6 mice. (d) Covered with BMMSC/gelfoam/aspirin. (e) The calvarial bone defect was sutured.

### SUMMARY OF THE INVENTION

The present invention discloses methods and compositions for improved BMMSC-based tissue engineering through suppression of IFN-γ and TNF-α, preferably by site-specific treatment and more preferably by treating with aspirin.

Proinflammatory T cells contribute to the inhibition of BBMSC-based bone formation. It is a discovery of the present invention that the cytokines IFN-γ and TNF-α play a major role in proinflammatory T cell-mediated suppression on BMMSC-based bone formation. In particular, the present invention has discovered that high levels of IFN-γ and TNF-α in BMMSC implants are negatively with bone formation. In contrast, blockage of IFN-γ and TNF-α with their respective neutralizing antibodies was effective in rescuing BMMSC-mediated bone formation. For example, addition of proinflammatory cytokine reducing agent(s), such as aspirin (e.g. 100 µg) to BMMSC implants restored bone formation that was suppressed by IFN-γ and TNF-α.

Accordingly, one aspect of the present invention is directed to a tissue engineering complex composition or a tissue regeneration device to be applied to a site in need of tissue regeneration. Compositions or devices in accordance with this aspect of the invention will generally include BMMSCs and proinflammatory cytokine reducing agent(s) in an amount effective to reduce the level of the proinflammatory cytokines. In some embodiments, the compositions or devices may further include a substrate. In still some other embodiments, the compositions or devices may further include at least one carrier.

In the context of the present invention, the terms "proinflammatory cytokine reducing agent" and "anti-inflammatory agent" are used interchangeably. They may be any agents capable of directly or indirectly reducing the effect of proinflammatory cytokine IFN-γ or TNF-α, or both. For example, antibodies capable of neutralizing these cytokines may be used as the anti-inflammatory agents. Any other known agents or future developed agents capable of inhibiting or down-regulating these cytokines may also be used as the anti-inflammatory agent of the present invention. In a preferred embodiment, the proinflammatory cytokine is aspirin. In another preferred embodiment, the amount of aspirin is preferably from about 50 µg/ml to about 100 µg/ml, more preferably 50 µg/ml.

Carrier(s) suitable for use in the compositions or devices of the invention is preferably a liquid such as Extracel® Hydrogel.

Substrates suitable for use in the compositions or devices of the invention is preferably water-insoluble, nonelastic, porous, pliable, and able to absorb or adsorb the BMMSCs and the anti-inflammatory agent(s). In a preferred embodiment, the substrate is a sponge such as GEL-FOAM®. Other materials known in the art with comparable properties may also be used. One example for this purpose is absorbable gelatin sponge.

Another aspect of the present invention is directed to a method of forming a composition or device as described above. Methods in accordance with this aspect of the invention will generally include the steps of adding an effective amount of one or more anti-inflammatory agent(s) to BMMSC. In some embodiments, a step of adding the anti-inflammatory agent(s) and/or BMMSCs to a carrier may be included. In some other embodiments, a step of depositing the anti-inflammatory agents and/or the BMMSCs onto a substrate may also be included. In one preferred embodiment, to form the composition/device, BMMSCs may be treated with aspirin for a suitable amount of time, preferably about 3 days, and mixed with hydrogel. Such hydrogel-BMMSCs may be transplanted with aspirin into newly generated calvarial bone defects. Another part of BMMSCs may be seeded to GEL-FOAM® and cultured for suitable amount of time, preferably about 3 days, with aspirin. The resulting gelfoam-BMMSCs containing aspirin may be used to cover the previously implanted BMMSC-hydrogel complex in the calvarial bone defect area.

Another aspect of the present disclosure is directed to a method of improving tissue regeneration at a site in need of new tissue. Methods in accordance with this aspect of the invention will generally include the step of applying a complex composition or device as described above to the site. Sites in need of tissue regeneration may be any site accessible either directly or indirectly. For example, it may be an *ex vivo* site or an *in vivo* site. In one preferred embodiment, the site is on an artificially grown tissue/organ. In another preferred embodiment, the site is calvarial defect site.

Another aspect of the present disclosure is directed to a method of improving BMMSC-mediated bone tissue formation in a subject. Methods in accordance with this aspect of the present invention will generally include the step of infusing a Foxp3+ regulatory T cells (Tregs) systemically to the subject for a predetermined duration prior to applying a BMMSC-based tissue regeneration inducer to the subject. In some embodiments, the BMMSC-based tissue regeneration inducer may be just seed BMMSCs. In other embodiments, the BMMSC-based tissue regeneration inducer may be BMMSCs in a carrier. In still some other embodiments, the BMMSC-based tissue regeneration inducer may be a tissue regeneration composition or device as described above.

While the above described embodiment outlines the general steps and elements of the present invention, it will be understood by those skilled in the art that various modifications are possible. Other aspects and advantages of the present invention will become apparent from the following detailed description and the appended claims.

### DETAILED DESCRIPTION

### Abbreviations:

As used herein, the abbreviation "BMMSC" stands for "Bone marrow mesenchymal stem cell".

As used herein, the abbreviation "Th1" stands for "T helper 1".

As used herein, the abbreviation "Runx-2" stands for "Runt-related transcription factor 2".

As used herein, the abbreviation "TNF-α" stands for "Tumor necrosis factor α".

As used herein, the abbreviation "NFκB" stands for "Nuclear factor kappa B".

As used herein, the abbreviation "Tregs" stands for "regulatory T cells".

Bone marrow mesenchymal stem cells (BMMSCs) are non-hematopoietic multipotent stem cells capable of differentiating into both Mesenchymal and non-mesenchymal cell types, including osteoblasts, adipocytes, and chondrocytes. When implanted *in vivo*, BMMSCs can form bones and induce recipient cells to form hematopoietic marrow components.

It is one discovery of the present invention that IFN-γ and TNF-α play critical roles in governing BMMSC-based bone formation *in vivo.* An increase in IFN-γ and TNF-α is negatively correlated with BMMSC-mediated bone formation in the BMMSC implants. IFN-γ synergistically enhances TNF-α-induced BMMSC apoptosis and inhibits osteogenesis of BMMSCs.

Without being limited to theory, our data suggests that Fas signaling is required for the IFN-γ synergistic effect on TNF-α-induced BMMSC apoptosis. Fas internalization and clustering apparently contribute to synergistic BMMSC apoptosis via activation of caspase 8/3 in the presence of IFN-γ and TNF-α. IFN-γ treatment activated Fas expression levels in BMMSCs to initiate a non-apoptotic pathway, in which osteogenesis was inhibited by activation of Smad 6 and reduction of Runx2. Treatment of BMMSCs with IFN-γ and TNF-α together, however, was able to convert the non-apoptotic Fas signaling to a death pathway *via* inhibition of TNFR2/NFκB alternative signaling pathway.

In short, inhibition of IFN-γ and TNF-α improves BMMSC-based bone regeneration. Treg treatment also improves BMMSC-based tissue engineering for the repair of calvarial defects. Infusion of Tregs into a recipient prior to BMMSC implantation result in complete repair of the defects as assessed by microQCT (**Figure 6D** and **6E**). The regenerated bone structures were identical to the original calvarial structures as assessed by microQCT and histological analysis (**Figure 6A, 6D,** and **6F**). Site-specific aspirin treatment improved BMMSC-based repair of calvarial defects via inhibition of IFN-γ and TNF-α. Site-specific pharmacological treatment methods inhibit IFN-γ and TNF-α expression and improve BMMSC-based calvarial defect repairing. Addition of aspirin (100 µg) to BMMSC implants restored bone formation that was suppressed by IFN-γ or TNF-α.

Accordingly, one aspect is directed to an improved method for BMMSC-mediated tissue regeneration. Methods in accordance with this aspect of the invention preferably includes administering to a subject in need thereof an effective amount of a composition in an amount sufficient to reduce or inhibit a proinflammatory cytokine selected from the group consisting of IFN-γ, TNF-α, and both.

Another aspect is directed to an improved method of bone regeneration which comprises reducing the levels of an anti-inflammatory agent(s) selected from the group consisting of IFN-γ, TNF-α, and both. Methods in accordance with this aspect of the invention preferably includes depositing (or placing) BMMSC's at a particular site or environment, and administering the composition locally at the site and or environment where the BMMSCs have been deposited.

Yet another aspect of the present invention is directed to a complex compositions or a device. Complex compositions or devices in accordance with this aspect of the invention generally includes agents or pharmaceutical compositions capable of reducing the level of IFN-γ, TNF-α, or preferably both, more preferably in BMMSC deposition sites. The complex compositions may also be pharmaceutical compositions, preferably a pharmaceutical composition including aspirin. More preferably, the pharmaceutical composition includes at least one of Tregs and aspirin.

Local administration of aspirin reduces levels of IFN-γ and TNF-α in the implantation site and significantly improves BMMSC-based calvarial defect repairing. These data collectively uncover a previously unrecognized role of recipient T cells in BMMSC-based tissue engineering and suggest a practical approach for enhancing bone regeneration by pharmacological control of local cytokines. The invention should be understood as including aspirin and other structurally or functionally related compounds known to reduce levels of levels of IFN-γ and TNF-α.

In other words, we have developed a practical approach to improve BMMSC-based tissue engineering through suppression of IFN-γ and TNF-α by a site-specific aspirin treatment. By adoptive transfer of T cells into immunocompromised mice, we determined that proinflammatory T cells contribute to the inhibition of BMMSC-based bone formation. We further identified that IFN-γ and TNF-α played a major role in proinflammatory T cell-mediated suppression on BMMSC-based bone formation. While not intending to be bound by any particular theory, the discoveries of the present invention may be explained by the finding that high levels of IFN-γ and TNF-α in BMMSC implants were negatively correlated with bone formation and blockage of IFN-γ and TNF-α with their respective neutralizing antibodies was effective in rescuing BMMSC-mediated bone formation.

It is unknown how IFN-γ elevates Fas expression level in BMMSCs, this appeared the case occurred in osteosarcoma cell lines (6). Interestingly, IFN-γ-induced Fas expression associated with IFN-γ-mediated blockage of osteogenic differentiation of BMMSCs by activating Smad-6 pathway. It was known that Smad-6 inhibits osteogenic differentiation via BMP and runx2 signaling (7, 8). However, a relatively high concentration of IFN-γ was required to be effective in inhibiting the osteogenesis of BMMSCs. Therefore, the inhibitory function of IFN-γ in BMMSC-mediated bone formation was likely due to its synergistic effect with TNF-α, which resulted in enhanced BMMSC apoptosis by activation of Fas signaling-mediated death pathway. In this regard, IFN-γ treatment alone can up-regulate Fas expression, but failed to induce BMMSC apoptosis, which might be attributed to the anti-apoptotic function of TNFR2-associated NFκB, XIAP, and FLIP in this cascade (9, 10). With the addition of TNF-α treatment, however, IFN-γ-induced non-apoptotic Fas signaling was converted to an apoptotic cascade due to the reduction of anti-apoptotic factors NFκB, XIAP, and FLIP as evidenced by Fas internalization and activation of death mediators caspase 3 and 8.

BMMSCs, including autologous BMMSCs, failed to regenerate bone structures in C57BL6 mice when implanted subcutaneously, which is likely associated with Th1 cytokine IFN-γ and TNF-α. The same BMMSC implantation easily regenerates bone in T cell deficient immunocompromised mice. In line with this observation, infusion of Tregs reduced IFN-γ and TNF-α levels and enhanced BMMSC-mediated bone formation. Importantly, a calvarial bone defect model was used to validate the efficacy of BMMSC-based tissue engineering as it represents a clinically relevant bone defect model and easily evaluate the quantity and quality of newly regenerated bone (11, 12).

Aspirin is a widely used nonsteroidal anti-inflammatory agent (NSAID). It inhibits osteoclastogenesis, and improves osteogenesis by affecting multiple biological pathways, such as inhibition of COX2, COX1, and PGE2 activity (13-15). In the present invention, we unexpectedly discovered that aspirin suppressed TNF-α and IFN-γ levels and reversed the proinflammatory cytokine-induced osteogenic deficiency of BMMSCs. We further used subcutaneous implantation and a calvarial bone defect repairing model to show that aspirin treatment is an appropriate approach for improving BMMSC-based tissue regeneration by suppression of IFN-γ and TNF-α.

### Description of Certain Findings and Embodiments

Recipient T cells modulate BMMSC-mediated bone regeneration *via* IFN-γ and TNF-α. Without being limited to any particular theory, we hypothesized that T cells modulate implanted donor BMMSCs. Using an established *in vivo* BMMSC implantation system, in which 4×10⁶ BMMSCs with carrier hydroxyapatite tricalcium phosphate (HA/TCP) particles were subcutaneously implanted into C57BL6 or immunocompromised mice (**Figure 1A**), we showed that BMMSCs derived from the littermate were unable to generate bone structures in C57BL6 mice at 8 weeks post implantation (**Figure 1B**). The same results were observed with autologous BMMSC implantation (**Figure 8**). In contrast, when BMMSCs were implanted into T cell-deficient immunocompromised mice, they formed bone and associated hematopoietic marrows (**Figure 1C**). To examine whether recipient T cells affected BMMSC-mediated bone formation, we infused 1×10⁶ pan-T cells into immunocompromised mice 2 days prior to subcutaneous BMMSC implantation. The BMMSC-mediated bone formation was completely blocked in these T cell-infused immunocompromised mice (**Figure 1**D). Only connective tissue around HA/TCP particles were observed in the BMMSC implants, similar to that of BMMSC implants in C57BL6 mice (**Figure 1B**). These data indicate that recipient T cells may play a crucial role in inhibiting BMMSC-mediated bone formation.

Next, we examined if specific subsets of T cells block BMMSC-based bone regeneration. We found that infusion of 1×10⁶ CD4⁺ or CD4⁺CD25⁻ T cells into immunocompromised mice intravenously blocked BMMSC-mediated bone formation (**Figures 1E** and **1F**). However, administration of CD4⁺CD25⁺Foxp3⁺ cells (Tregs) showed no inhibitive effect on BMMSC-mediated bone formation in immunocompromised mice (**Figures 1G** and **1H**), suggesting that non-Treg cells inhibit BMMSC-mediated bone formation.

To explore mechanisms of T cell-regulated BMMSC-based bone formation, we assessed the levels of a variety of cytokines, including IFN-γ, TNF-α, IL-4, IL-6, and IL-17A in the BMMSC implants (**Figures 1I-M**). Interestingly, the levels of IFN-γ and TNF-α in BMMSC implants were significantly elevated at 7-14 days after BMMSC implantation in C57BL6 mice and in immunocompromised mice receiving pan-T cell or CD4⁺CD25⁻ T cells (**Figures 1I, 1K, 1L**)**.** The BMMSC implants with high levels of IFN-γ and TNF-α were correlated with lack of new bone formation at 8 weeks after implantation (**Figures 1B, 1D, 1E, 1F****,** **1H**). Conversely, the levels of above mentioned cytokines were not altered in the BMMSC implants derived from immunocompromised and Treg-infused immunocompromised mice after 2-21 days implantation (**Figures 1J and 1M**). These implants showed substantial amount of new bone formation (**Figures 1C and 1G**). Thus, the increase in IFN-γ and TNF-α is negatively correlated with BMMSC-mediated bone formation in the BMMSC implants.

To further assess the role of the cytokines in BMMSC-mediated bone formation, 200 ng of IFN-γ, TNF-α, IL-4, IL-6, or IL-17A was co-implanted with BMMSCs into immunocompromised mice subcutaneously (**Figures 1N-T**). IFN-γ or TNF-α treatment reduced BMMSC-mediated bone formation at 8 weeks post implantation (**Figures 1R and 1S**). Meanwhile, IL-4 or IL-6 treatment slightly inhibited BMMSC-mediated bone formation (**Figures 1O and 1P**), and IL-17A treatment had no inhibitory effect on BMMSC-mediated bone formation (**Figure 1Q**) when compared to the un-treated control group (**Figures IN and 1T**). In contrast, injection of IFN-γ and TNF-α neutralizing antibodies substantially rescued BMMSC-mediated bone formation in C57BL6 mice (**Figures 1U-W**). Thus, both IFN-γ and TNF-α play critical roles in governing BMMSC-based bone formation *in vivo.*

CD4⁺CD25⁺Foxp3⁺ Tregs inhibit T cell activation and reduce IFN-γ and TNF-α production. Thus, we infused 1×10⁶ Tregs into C57BL6 mice 2 days prior to the BMMSC implantation and found substantial amount of bone formation in Treg-infused BMMSC implants and lack of bone formation in control BMMSC implants (**Figure 2A-****2C**). To examine whether the protective role of Tregs in BMMSC-based bone formation was attributable to the suppression of proinflammatory cytokines in C57BL6 mice, we measured the cytokine levels in the Tregs-treated mice. We observed that Tregs infusion reduced the levels of IFN-γ and TNF-α, but not the levels of IL-4 and IL-10 (**Figure 2D**) in BMMSC implants. Thus, inhibition of IFN-γ and TNF-α improves BMMSC-based bone regeneration.

IFN-γ inhibited osteogenesis of BMMSCs. When IFN-γ T cells were systemically infused into immunocompromised mice, they failed to block BMMSC-mediated bone formation (**Figure 9**), suggesting that IFN-γ is required for T cell-mediated osteogenic inhibition (**Figures 1D** **and** **1K**). Further analysis showed that *in vitro* IFN-γ (200 ng/ml), but not IL-6 or IL-17A, treatment inhibited osteogenic differentiation of BMMSCs compared to untreated-control groups as evidenced by alizarin red staining (**Figures 3A**). IL-4 treatment (200 ng/ml) served as a positive control showing a partial inhibition of BMMSC osteogenic differentiation (24; **Figures 3A**). Interestingly, 50 ng/ml IFN-γ treatment had no inhibitory effect on osteogenic differentiation of BMMSCs (**Figure 3B**). This dose-dependent osteogenic inhibition by IFN-γ was associated with up-regulated expression of Smad 6, a negative regulator of osteogenic differentiation, and down-regulated expression of the ostegenic genes runt-related transcription factor 2 (Runx2), osteocalcin, and alkaline phosphatase (ALP) in 200 ng/ml IFN-γ group, which were not observed in 50 ng/ml IFN-γ group (**Figure 3C**).

Furthermore, we revealed that IFN-γ treatment activated Fas expression in BMMSCs (**Figure 4F**) and IFN-γ treatment failed to reduce ostoegenic differentiation and alter expression levels of Smad 6 and Runx2 in Fas^{-/-} BMMSCs (**Figure 3D**) and Fas knock down BMMSCs by siRNA (**Figure 3E**).

IFN-γ synergistically enhanced TNF-α-induced BMMSC apoptosis. When BMMSCs were cultured with a variety of cytokines, including IFN-γ, TNF-α, IL-4, IL-6, IL-17A, and TGF-β, only TNF-α treatment resulted in significant cell death in a dose-dependent manner as evidenced by toluidine blue staining (**Figure 4A**). To determine effect of IFN-γ on TNF-α-treated BMMSCs, we added 50 ng/ml IFN-γ to BMMSCs that were treated with different doses of TNF-α (1-200 ng/ml; Figure 4B). IFN-γ treatment significantly enhanced BMMSC apoptosis when the TNF-α concentration reached 50-200 ng/ml as assessed by toluidine blue staining (**Figure 4B**) and annexin V apoptosis flow cytometric analysis (**Figure 10**). To further confirm the synergistic effects of TNF-α and IFN-γ on the induction of BMMSC apoptosis, we added 50 ng/ml TNF-α to BMMSCs that were treated with 1-200 ng/ml IFN-γ (**Figure 4C**). The amount of BMMSC apoptosis was correlated with IFN-γ concentration from 1-50 ng/ml. When IFN-γ concentration reached to 100-200 ng/ml, a complete BMMSC apoptosis was observed (**Figure 4C**). In addition, we used immunocytostaining with annexin V to confirmed that 50 ng/ml IFN-γ enhanced 20 ng/ml TNF-α-mediated BMMSC apoptosis (**Figure 4D**). Next, we verified the IFN-γ synergic effect on TNF-α-induced BMMSC apoptosis using an *in vivo* subcutaneous GFP⁺ BMMSC implantation model (16, 17). Thirty days after implantation, GFP⁺ BMMSCs pre-treated with 50 ng/ml TNF-α showed a significant reduction in the numbers of surviving cells than those from untreated control group (**Figure 4E**). Combination treatment with 200 ng/ml IFN-γ and 50 ng/ml TNF-α led to no survival of GFP⁺ BMMSCs in the implants (**Figure 4E**). IFN-γ is capable of synergistically enhancing TNF-α-induced BMMSC apoptosis.

We next examined the molecular mechanism by which IFN-γ enhanced TNF-α-induced BMMSC apoptosis. We found that IFN-γ treatment up-regulated Fas expression in BMMSCs without activating caspase 3 and caspase 8 (**Figure 4F**), suggesting that IFN-γ-induced Fas expression was not capable of initiating the apoptotic process. With the addition of 20 ng/ml TNF-α, a concentration unable to induce marked BMMSC apoptosis, to 50 ng/ml IFN-γ-treated BMMSCs, caspase 3 and caspase 8 were activated (**Figure 4F**) along with significant BMMSC apoptosis (**Figure 4H**). However, combination of 20 ng/ml TNF-α and 50 ng/ml IFN-γ treatment failed to activate caspase 3 and caspase 8 in Fas^{-/-} BMMSCs (**Figure 4F**). To further confirm that Fas signaling contributes to accelerated apoptotic process in IFN-γ/TNF-α-treated BMMSCs, we used siRNA to knockdown Fas expression in BMMSCs and showed that activated caspase 8 and caspase 3 were partially blocked in IFN-γ/TNF-α treated BMMSCs (**Figure 4G**) with a significantly reduction in the number of apoptotic BMMSCs (**Figure 4H**). These data suggest that Fas signaling is required for the IFN-γ synergistic effect on TNF-α-induced BMMSC apoptosis.

Next question we asked is how Fas apoptotic signaling was activated in IFN-γ/TNF-α-treated BMMSCs. We revealed that knockdown Fas-L level using siRNA in IFN-γ/TNF-α-treated BMMSCs was not able to reduced number of apoptotic cells (data not shown), excluding Fas-L as a potential factor contributing to BMMSC apoptosis. Thus, we hypothesized that Fas internalization might activate Fas/caspase 8/3 apoptotic signaling in IFN-γ/TNF-α-treated BMMSCs. Using fluorescent immunocytostaining, we showed that combined treatment of IFN-γ and TNF-α induced Fas internalization and clustering. In un-treated control group, Fas was evenly distributed on BMMSC surface (**Figure 5A**). BMMSCs were treated with IFN-γ for 12 hrs and then TNF-α was added, Fas showed marked clustering and internalization in cytoplasm at 0.5-1 hr (**Figure 5A**). More significant Fas clustering with shrinkage of the cell and cell membrane broken down was observed at 2-4 hrs TNF-α-treatment (**Figure 5A**). Blockage of Fas internalization by endocytosis inhibitor Latrunadin A (LntA) could partially rescue IFN-γ/TNF-α-induced BMMSC apoptosis with inhibition of cleaved caspase 3 and 8 (**Figure 5B**). Interestingly, with blocking caspase 8 or caspase 3 activities using their specific inhibitors, IFN-γ/TNF-α-treated BMMSCs exhibited significant reduction in the number of apoptotic cells (**Figure 5C**). Moreover, we showed that inhibition of pan caspase, caspase 3, and caspase 8 was able to partially block Fas internalization as shown by immunostaining (**Figure 5D**). These data suggest that Fas internalization and clustering contribute to synergistic BMMSC apoptosis via activation of caspase 8/3 in the presence of IFN-γ and TNF-α.

We next investigate how IFN-γ and TNF-α together resulted in Fas internalization and clustering in BMMSCs. First, we showed that IFN-γ/TNF-α treatment could not induce significantly up-regulated expression of TNFR1, TRAIL, and Fas L proteins (**Figure 11**). Then we found that IFN-γ/TNF-α treatment reduced levels of TNFR2 and its downstream signaling phosphorylated NFκB, XIAP, and FLIP when compared to either IFN-γ or TNF-α treated group (**Figure 5E**), suggesting that combination of IFN-γ and TNF-α treatment selectively inhibit TNFR2 pathway that provides anti-apoptotic effect. To confirm the role of TNFR2 pathway in IFN-γ/TNF-α-induced BMMSC apoptosis, we showed that reduction of TNFR2, IKK, XIAP, and FLIP by siRNA further activated caspase 8 and 3 (**Figure 5F**). The efficiency of TNFR2, IKK, XIAP, and FLIP knockdown by their specific siRNAs was confirmed by Western blot analysis (**Figure 5G**).

These findings collectively revealed that IFN-γ treatment activated Fas expression levels in BMMSCs to initiate a non-apoptotic pathway, in which osteogenesis was inhibited by activation of Smad 6 and reduction of Runx-2 (**Figure 12A**). Treatment of BMMSCs with IFN-γ and TNF-α together, however, was able to convert the non-apoptotic Fas signaling to a death pathway via inhibition of TNFR2/NFκB alternative signaling pathway (**Figure 12B**).

Treg treatment improves BMMSC-based tissue engineering for the repair of calvarial defects. Since infusion of Tregs inhibited levels of TNF-α and IFN-γ in the implantation site of C57BL6 mice (**Figure 2D**), we hypothesized that Treg treatment could improve cell-based tissue engineering. To test this hypothesis, we generated critical-sized calvarial bone defects (7×8 mm) in C57BL6 mice (**Figures 6A and 6B**). Very small amounts of bone formation were observed in the untreated group at 12 weeks after surgery (**Figure 6B**). Implantation of BMMSCs with gelfoam as a carrier showed moderate bone regeneration, but failed to completely repair the defects (**Figures 6C**). However, infusion of Tregs into the recipients 2 days prior to BMMSC/gelfoam implantation resulted in complete repair of the defects as assessed by microQCT (**Figure 6D and 6E**). The regenerated bone structures were identical to the original calvarial structures as assessed by microQCT and histological analysis (**Figure 6A, 6D, and 6F**). These data imply that Treg treatment improves BMMSC-mediated tissue engineering.

Site-specific aspirin treatment improved BMMSC-based repair of calvarial defects *via* inhibition of IFN-γ and TNF-α. Since aspirin may inhibit the function of TNF-α and IFN-γ (14), we examined the effect of aspirin treatment on IFN-γ and TNF-α levels in BMMSC implants at 2-14 days after implantation. We showed that aspirin treatment reduced the levels of IFN-γ and TNF-α without affecting IL-10 level (**Figure 7A**). Immunohistochemical analysis confirmed the suppressive effect of aspirin treatment on the numbers of IFN-γ and TNF-α positive cells in BMMSC implants (**Figures 7B-I****).** To determine if aspirin-mediated reduction of IFN-γ or TNF-α in BMMSC implants influenced the apoptosis of BMMSCs, we utilized a GFP⁺ BMMSC implantation model to show a significant increase in survival of BMMSCs in aspirin-treated group during 4-30 days post implantation (**Figure 13A**).

Next, we studied the effect of aspirin on inhibition of exogenous IFN-γ and TNF-α on BMMSC-mediated bone formation *in vivo.* We treated BMMSCs with 50 µg/ml aspirin for 2 days followed by subcutaneous implantation into immunocompromised mice using HA/TCP as a carrier. The aspirin pre-treatment increased BMMSC-mediated bone formation compared to the untreated control group (**Figure 13B, 13C**). We then implanted BMMSC with IFN-γ or TNF-α (200 ng) and observed a significantly reduced bone formation (**Figures 1R-T** **and** **Figures 13D and 13E**). However, addition of aspirin (100 µg) to BMMSC implants restored bone formation that was suppressed by IFN-γ or TNF-α (Figures 13F- 13H). Moreover, we investigated whether aspirin treatment could be applied to enhance BMMSC-based calvarial defect repairing. Indeed, implantation of BMMSCs (4×10⁶) with gelfoam containing aspirin (100 µg) resulted in a marked improvement in repairing calvarial defects compared to the control BMMSC/gelfoam group (**Figures 7J and 7K**). The structures of regenerated tissues were analogous to normal calvarial tissues (**Figures 7L-7N**).

### EXPERIMENTAL

### Material and Methods

**Animals.** Female C3H/HeJ, C57BL/6J, B6.129S7-*Ifng*^{*tmlTs*/}J, C57BL/6-Tg(CAG-EGFP)1Osb/J, B6.MRL-*Fas^{lpr}*/J mice were purchased from Jackson Lab (Bar Harbor, ME). Female immunocompromised mice (Beige *nude*/*nude* XIDIII) were purchased from Harlan (Indianapolis, IN). All animal experiments were performed under the institutionally approved protocols for the use of animal research (University of Southern California protocols #10874 and 10941).

**Antibodies and inhibitors.** Unconjugated anti-TNF-α, anti-Fas, anti-Smad 6 and anti-Smad 7 antibodies were purchased from Santa Cruz Biosciences (Santa Cruz, CA). Unconjugated anti-GFP, anti-OCN, anti-TRAIL, anti-TNFR1, anti-FasL, anti-FLIP, and anti-XIAP antibodies were purchased from Abcam Inc. (San Francisco, CA). Unconjugated anti-Runx-2 was purchased from Calbiochem Inc. (La Jolla, CA). Anti-TNFR2, anti-caspase 8, anti-caspase 3, anti-cleaved casepase 8 and anti-cleaved caspase 3 antibodies were purchased from Cell Signaling Inc. (San Francisco, CA). Anti-IFN-γ-FITC antibody was purchased from eBioscience (San Diego, CA). Anti β-actin antibody was purchased from Sigma (St Louis, MO). LEAF™ Purified anti-mouse IFN-γ antibody and anti-mouse TNF-α antibody were purchased from Biolegend (Danvers, MA). Recombinant murine IFN-γ, TNF-α, IL-6, IL-17A, IL-4 were purchased from PeproTech (Rocky Hill, NJ). Alexa Fluor® 488 phalloidin was purchased from Invitrogen Corporation (Carlsbad, CA). Pan caspase inhibitor I (Z-VAD (OMe)-FMK) was purchased from EMD Chemicals, Inc (Gibbstown, NJ). Inhibitors for caspase 8 (2-IETD-FMK) and caspase 3 (Z-DEVD-FMK) were purchased from R&D System (Minneapolis, MN). Inhibitor for internalization (Latrunculin A) was purchased from Sigma-Aldrich Corporate (St. Louis, MO). HyStem™ and Extracel-HP hydrogel was purchased from Glycosan (Salt Lake City, Utah).

**Kits.** Pan T cell isolation kit II and CD4⁺CD25⁺ regulatory T Cell isolation kit were purchased from Miltenyi Biotec (Bergisch Gladbach, Germany). Mouse IFN-γ, TNF-α, IL-6, IL-17A, IL-4 and IL-10 ELISA Ready-SET-GO kits were purchased from eBioscience (San Diego, CA). siRNA kits for Fas, Smad 6, TNFR2, IKK, FLIP and XIAP were purchased from Santa Cruz Biosciences (Santa Cruz, CA).

**Isolation of mouse bone marrow Mesenchymal stem cells (BMMSCs).** Bone marrow cells were flashed out from bone cavity of femurs and tibias with 2% heat-inactivated fetal bovine serum (FBS; Equitech-Bio, Kerrville, TX) in PBS. Single-cell suspension of all nuclear cells (ANCs) was obtained by passing through 70 µm cell strainer (BD Bioscience, Franklin Lakes, NJ). CD146 positive ANCs were sorted out and seeded at 1×10⁴ into 100 mm culture dishes (Corning Corporation, Corning, NY) and initially incubated for 48 hours under 37 °C at 5% CO₂ condition. To eliminate the non-adherent cells, the cultures were washed with PBS twice. The attached cells were cultured for 16 days. The BMMSCs were cultured with alpha minimum essential medium (α-MEM, Invitrogen Corporation, Carlsbad, CA) supplemented with 20% FBS, 2 mM L-glutamine (Invitrogen Corporation), 55 µM 2-mercaptoethanol (Invitrogen Corporation), 100 U/ml penicillin, and 100 µg/ml streptomycin (Invitrogen Corporation).

**Immunofluorescent and immunochemical microscopy.** The transplants were harvested, fixed in 4% paraformaldehyde and then decalcified with 5 % ethylenediaminetetraacetic acid (EDTA, pH 7.4), followed by paraffin or optimal cutting temperature compound (OCT, Sakura Fineteck Inc., Torrance, CA) embedding. The paraffin or frozen sections were blocked with normal serum matched to secondary antibodies, incubated with the specific or isotype-matched mouse antibodies (1:200) overnight at 4°C. For immunofluorescent staining, the samples were treated with Rhodamin/FITC-conjugated secondary antibodies (1:200, Jackson ImmunoResearch, West Grove, PA; Southern Biotechnology, Birmingham, AL), and were mounted by means of a vectashield mounting medium containing 4',6-diamidino-2-phenylindole (DAPI) (Vector Laboratories, Burlingame, CA). For immunochemical staining, the samples were stained by using Zymed SuperPicture™ Kit (Invitrogen Corporation) according to the manufacturer's instruction.

**BMMSC-mediated bone formation.** Approximately 4.0×10⁶ BMMSCs were mixed with hydroxyapatite/tricalcium phosphate (HA/TCP) ceramic particles (40 mg, Zimmer Inc., Warsaw, IN) as a carrier and subcutaneously implanted into the dorsal surface of 8-10 weeks old immunocompromised mice or C57BL/6J mice. At eight weeks post-implantation, the implants were harvested, fixed in 4% paraformaldehyde and then decalcified with 5 % EDTA (pH 7.4), followed by paraffin embedding. The 6 µm paraffin sections were stained with hematoxylin and eosin (H&E) and analyzed by an NIH Image J. Five fields were selected and newly formed mineralized tissue area in each field was calculated and shown as a percentage to total tissue area.

**Cytokines in BMMSC-mediated bone formation.** Approximately 4.0×10⁶ of littermate-derived or autologous BMMSCs were mixed with HA/TCP ceramic powders (40 mg, Zimmer Inc.), and hydrogel (Glycosan Biosysterms, Salt Lake City, UT) with 200 ng IFN-γ, TNF-α, IL-4, IL-6 or IL-17A were covered on the surface of the implants as a slow released system. At eight weeks post-implantation, the implants were harvested and newly formed mineralized tissue areas were analyzed.

**Osteogenic differentiation assay.** BMMSCs were cultured under osteogenic culture condition media containing 2 mM β-glycerophosphate (Sigma-Aldrich), 100 µM L-ascorbic acid 2-phosphate and 10 nM dexamethasone (Sigma-Aldrich). Different doses of IFN-γ (50, 200 ng/ ml) were added to the osteogenic culture condition media every 3 days. After the osteogenic induction, the cultures were stained with alizarin red. Expressions of Runx2, ALP, and OCN were assayed by Western blot analysis.

***In vivo* cell infusion.** Approximately 1.0×10⁶ of cells were suspended in 200 µl PBS and injected into the mice *via* tail vein.

**Western blot analysis.** Total protein was extracted using M-PER mammalian protein extraction reagent (Thermo, Rockford, IL). Nuclear protein was obtained using NE-PER nuclear and cytoplasmic extraction reagent (Thermo). Protein was applied and separated on 4-12% NuPAGE gel (Invitrogen) and transferred to Immobilon™-P membranes (Millipore). The membranes were blocked with 5% non-fat dry milk and 0.1% tween-20 for 1 hour, followed by incubation with the primary antibodies (1:200-1000 dilution) at 4°C overnight. Horseradish peroxidase-conjugated IgG (Santa Cruz Biosciences; 1:10,000) was used to treat the membranes for 1 hour, and enhanced with a SuperSignal® West Pico Chemiluminescent Substrate (Thermo). The bands were detected on BIOMAX MR films (Kodak, Rochester, NY). Each membrane was also stripped using a stripping buffer (Thermo) and reprobed with anti-β-actin antibody to quantify the amount of loaded protein.

**Cell apoptosis and cell survival assay.** 0.5×10⁶ BMMSCs were seeded to 6-well culture plate and co-cultured with different doses of recombined IL-4, IL-6, IL-17A, TGF-β1, TNF-α, and IFN-γ (0, 10, 20, 50, 100, 200 ng/ml) for 3 days. To measure the cell viability, the total cells were analyzed by using a cell counting kit-8 (Dojindo Molecular Technoloies, Gaithersburg, MD) according to the manufacture's instruction. And the culture plates with cells were stained with 2% toluidine blue O and 2% paraformaldehyde (Sigma-Aldrich Corporate, St. Louis, MO).

For cell apoptosis analysis, the BMMSCs were stained by Annexin V-PE apoptosis detection kit I (BD Bioscience) following manufacture's protocol, and analyzed by FACS^{Calibur} (BD Bioscience).

**IFN**-γ **and TNF-α synergism for apoptosis.** For cell apoptosis staining, the 1×10⁴ BMMSCs were seeded onto two chambers slide, IFN-γ (50 ng/ml) and TNF-α (20 ng/ml) were used to treat the BMMSCs for 24 hrs either separately or combined, Annexin V-PE apoptosis detection kit I (BD Bioscience) was used to stain the BMMSCs following manufacture's protocol, and analyzed on microscopy and FACSCalibur (BD Bioscience). Cell viability was analyzed by using cell counting kit, and culture plates were stained with 2% toluidine blue O and 2% paraformaldehyde.

**siRNA and Inhibitors** 0.5×10⁶ BMMSCs were seeded to 6-well culture plate. SiRNAs for Fas, TNFR2, IKK, FLIP and XIAP were used to treat the BMMSCs according to the manufacture's instruction, and IFN-γ and TNF-α were added for 24 hrs. Inhibitors for internalization (Latrunculin A, 0.2 µM), pan caspase (Z-VAD (OMe)-FMK, 50 µM), caspase 8 (2-IETD-FMK, 20 µM) and caspase 3 (Z-DEVD-FMK, 20 µM) were used to treat the BMMSCs at 4 hrs before IFN-γ and TNF-α were added. Cell apoptosis were analysis by immunofluorescence staining and FACS^{Calibur} (Annexin V-PE apoptosis detection kit I) and Western blot analysis for caspase 8, cleaved caspase 8, caspase 3 and cleaved caspase 3.

**Cell survival assay.** 4×10⁶ BMMSCs isolated from C57BL/6-Tg (CAG-EGFP)1Osb/J mice were combined with different concentrations of IFN-γ (0, 20, 50, 100, 200 ng/ml) and TNF-α (0, 20, 50, 100, 200 ng/ml) and subcutaneously transplanted into immunocompromised mice, hydrogel was used as a slowly released carrier. Thirty days after translation, the implants were harvested and digested in a solution of 3 mg/mL collagenase type I (Worthington Biochem) and 4 mg/mL dispase (Roche) for 1 h at 37 °C. Single-cell suspensions were obtained by passing the cells through a 70 µm strainer (BD Bioscience). 1.0-1.5×10⁶ BMMSCs were seeded into 100 mm culture dishes (Corning) and incubated for 48 hours under 37°C at 5% CO₂ condition. To eliminate the non-adherent cells, the cultures were washed with PBS twice. The attached cells were cultured for 10 days with alpha minimum essential medium (α-MEM, Invitrogen) supplemented with 20% FBS, 2 mM L-glutamine (Invitrogen), 55 µM 2-mercaptoethanol (Invitrogen), 100 U/ml penicillin, and 100 ng/ml streptomycin (Invitrogen). The numbers of GFP positive cells were counted under fluorescent microscopy.

**T lymphocyte isolation.** Pan T, CD4⁺, CD4⁺CD25⁻ T lymphocytes were isolated from mouse total spleen cells using a magnetic sorter, mouse Pan T cell isolation kit II and CD4⁺CD25⁺ regulatory T Cell isolation kit (Miltenyi Biotec, Bergisch Gladbach, Germany) following manufacture's instruction. The purity of the Pan T, CD4⁺, CD4⁺CD25⁻ was >95%.

**CD4⁺CD25⁺Foxp3⁺Tregs induction.** To induce CD4⁺CD25⁺Foxp3⁺Tregs *in vitro,* CD4⁺CD25⁻ T lymphocytes (1×10⁶/well) were collected by a CD4⁺CD25⁺ regulatory T cell Isolation kit (Miltenyi Biotec, Bergisch Gladbach, Germany) and 1×10⁶/well CD4⁺CD25⁻ T lymphocytes were cultured on 12 well multiplates in the presence of plate bounded anti CD3ε antibody (5 µg/ml), soluble anti CD28 antibody (2 µg/ml), recombinant mouse TGF-β1 (2 µg/ml) (R&D Systems) and recombinant mouse IL2 (2 µg/ml) (R&D Systems) for three days. All the wells were filled with a complete medium containing Dulbecco's Modified Eagle Medium (DMEM, Lonza, Walkersville, MD) with 10% heat-inactivated FBS, 50 µM 2-mercaptoethanol, 10 mM HEPES (Sigma), 1 mM Sodium pyruvate (Sigma), 1% non-essential amino acid (Cambrex, Charles City, IA), 2 mM L-glutamine, 100 U/ml penicillin and 100 mg/ml streptomycin. Then, CD4⁺CD25⁺ T lymphocytes were isolated using a magnetic sorter and mouse CD4⁺CD25⁺ regulatory T Cell isolation kit (Miltenyi Biotec, Bergisch Gladbach, Germany) following manufacture's instruction. The purity of CD4⁺CD25⁺Foxp3⁺ T lymphocytes was >95%, and they were used for systemic infusion.

**Fas internalization.** P1 BMMSCs were seeded to 2-well chamber slide. IFN-γ (50 ng/ml) was added to pre-treat BMMSCs for 24 hours, and then TNF-α (20 ng/ml) was added. At the 0.5, 1, 2, 4 hours after TNF-α was added, the cells were washed by PBS twice, and fixed with 4% PFA for 15 min. After treated by Triton-X 15 min, the cells were blocked with normal serum matched to secondary antibodies, incubated with 1 µg/ml anti-Fas (Santa Cruz) antibody at room temperature for 2 hours, treated with Rhodamin-conjugated secondary antibodies (1:200, Jackson ImmunoResearch). Then the cells were incubated with β-actin (1:40; Invitrogen) at room temperature for 20 min, and were mounted by vectashield mounting medium DAPI (Vector Laboratories).

**Calvarial bone defect model in C57BL/6J mice.** The skin of C57BL/6J mice was cut and periosteum was elevated. The surface of mouse cavarial bone was exposed, and oversize bone defects of 7 mm × 8 mm were established. The bone defect was larger than usually critical size with diameter of 4 mm.

**Implantation of BMMSCs to calvarial bone defects in C57BL/6J mice.** 4×10⁶ BMMSCs were seeded to a size of 7 mm × 8 mm gelfoam (Pharmaca, Boulder, CO) and cultured for 3 days *in vitro.* The gelfoam with BMMSCs was placed to the calvarial bone defects and covered completely by skins. For aspirin treated group, the BMMSCs seeded on gelfoam were pretreated with 50 µg/ml aspirin for 3 days, then placed to the calvarial bone defect. Another gelfoam with 100 µg aspirin as a releasing system was covered on the surface of the gelfoam with BMMSCs (Fig 14).

**Statistics.** SPSS 13.0 was used to do statistical analysis. Significance was assessed by independent two-tailed Student's *t*-test or analysis of variance (ANOVA). The *p* values less than 0.05 were considered significant.

**Isolation of mouse bone marrow Mesenchymal stem cells (BMMSCs).** Bone marrow cells were flashed out from bone cavity of femurs and tibias with 2% heat-inactivated fetal bovine serum (FBS; Equitech-Bio, Kerrville, TX) in PBS. Single-cell suspension of all nuclear cells (ANCs) was obtained by passing through 70 µm cell strainer (BD Bioscience, Franklin Lakes, NJ). CD146 positive ANCs were sorted out and seeded at 1×10⁴ into 100 mm culture dishes (Corning Corporation, Corning, NY) and initially incubated for 48 hours under 37 °C at 5% CO₂ condition. To eliminate the non-adherent cells, the cultures were washed with PBS twice at second day. The attached cells were cultured for 16 days. The BMMSCs were cultured with alpha minimum essential medium (α-MEM, Invitrogen Corporation, Carlsbad, CA) supplemented with 20% FBS, 2 mM Lglutamine (Invitrogen Corporation), 55 µM 2-mercaptoethanol (Invitrogen Corporation), 100 U/ml penicillin, and 100 µg/ml streptomycin (Invitrogen Corporation).

**BMMSC-mediated bone formation.** Approximately 4.0×10⁶ of littermate-derived or autologous BMMSCs were mixed with hydroxyapatite/tricalcium phosphate (HA/TCP) ceramic particles (40 mg, Zimmer Inc., Warsaw, IN) as a carrier and subcutaneously implanted into the dorsal surface of 8-10 weeks old immunocompromised mice or C57BL6 mice. When aspirin was used, BMMSCs were treated with 50 µg/ml aspirin for 2 days and mixed with HA/TCP particles to be subcutaneously implanted into immunocompromised mice. When IFN-γ and TNF-α were used in the subcutaneous implants, 200 ng IFN-γ, TNF-α or/and 100 µg aspirin mixed with 250 µl hydrogel as a slow released system and subcutaneously co-implanted to the dorsal surface of 8-10 weeks old immunocompromised mice.

At eight weeks post-implantation, the implants were harvested, fixed in 4% paraformaldehyde and then decalcified with 5 % EDTA (pH 7.4), followed by paraffin embedding. The 6 µm paraffin sections were stained with hematoxylin and eosin (H&E) and analyzed by an NIH Image J. Five fields were selected and newly formed mineralized tissue area in each field was calculated and shown as a percentage to total tissue area.

**Pan T cell isolation and infusion.** Pan T lymphocytes were isolated from mouse total spleen cells using a magnetic sorter, mouse Pan T cell isolation kit II. Approximately 1.0×10⁶ of cells were suspended in 200 µl PBS and injected into the mice *via* tail vein.

**Western blot analysis.** Total protein was extracted using M-PER mammalian protein extraction reagent (Thermo, Rockford, IL). Nuclear protein was obtained using NE-PER nuclear and cytoplasmic extraction reagent (Thermo). Protein was applied and separated on 4-12% NuPAGE gel (Invitrogen) and transferred to Immobilon™-P membranes (Millipore). The membranes were blocked with 5% non-fat dry milk and 0.1% tween-20 for 1 hour, followed by incubation with the primary antibodies (1:200-1000 dilution) at 4°C overnight. Horseradish peroxidase-conjugated IgG (Santa Cruz Biosciences; 1:10,000) was used to treat the membranes for 1 hour, and enhanced with a SuperSignal® West Pico Chemiluminescent Substrate (Thermo). The bands were detected on BIOMAX MR films (Kodak, Rochester, NY). Each membrane was also stripped using a stripping buffer (Thermo) and reprobed with anti-β-actin antibody to quantify the amount of loaded protein.

**IFN-γ and TNF-α synergism for apoptosis.** 0.5×10⁶ BMMSCs were seeded to 6-well culture plate and co-cultured with different doses of TNF-α (0, 5, 50, 100 ng/ml) with or without IFN-γ (50 ng/ml) for 1 day. Then, the BMMSCs were stained by Annexin V-PE apoptosis detection kit I (BD Bioscience) following manufacture's protocol, and analyzed by FACS^{calibur} (BD Bioscience).

**Immunochemical microscopy.** The transplants were harvested, fixed in 4% paraformaldehyde and then decalcified with 5 % ethylenediaminetetraacetic acid (EDTA, pH 7.4), followed by paraffin or optimal cutting temperature compound (OCT, Sakura Fineteck Inc., Torrance, CA) embedding. The paraffin or frozen sections were blocked with normal serum matched to secondary antibodies, incubated with the specific or isotype-matched mouse antibodies (1:200) overnight at 4°C. The samples were stained with Zymed SuperPicture™ Kit (Invitrogen Corporation) according to the manufacturer's instruction.

### REFERENCES

1. Kwan, M.D., Slater, B.J., Wan, D.C. & Longaker, M.T. Cell-based therapies for skeletal regenerative medicine. Hum Mol Genet 17, R93-98 (2008).
2. Panetta, N.J., Gupta, D.M., Quarto, N. & Longaker, M.T. Mesenchymal cells for skeletal tissue engineering. Panminerva Med 51, 25-41 (2009).
3. Fredericks, D.C. et al. Cellular interactions and bone healing responses to a novel porous tricalcium phosphate bone graft material. Orthopedics 27, s167-173 (2004).
4. Seong, J.M. et al. Stem cells in bone tissue engineering. Biomed Mater 5, 062001 (2010)
5. Undale, A.H., Westendorf, J.J., Yaszemski, M.J. & Khosla, S. Mesenchymal stem cells for bone repair and metabolic bone diseases. Mayo Clin Proc 84, 893-902 (2009).
6. Li, Z. et al. IFN-γ enhances HOS and U2OS cell lines susceptibility to T cell-mediated killing through the Fas/Fas ligand pathway. Int Immunopharmacol 11, 496-503 (2011)
7. Shen, R. et al. Smad6 intereacts with Runx2 and mediates Smad ubiquitin regulatory factor 1-induced Runx2 degradation. J Biol Chem 281, 3569-3576 (2006).
8. Itoh, F. et al. Promoting bone morphogenetic protein signaling through negative regulation of inhibitory Smads. EMBO J 20, 4132-4142 (2001).
9. Qin, J.Z. et al. Role of NF-κB in the apoptotic-resistant phenotype of keratinocytes. J Biol Chem 274, 37957-37964(1999).
10. Moon, D.O., Kim, M.O., Kang, S.H., Choi, Y.H. & Kim, G.Y. Sulforaphane suppresses TNF-alpha-mediated activation of NF-kappaB and induces apoptosis through activation of reactive oxygen species-dependent caspase-3. Cancer Lett 274, 132-142 (2009).
11. Koob, S. et al. Bone formation and neovascularization mediated by mesenchymal stem cells and endothelial cells in critical-sized calvarial defects. Tissue Eng Part A 17, 311-321(2011).
12. Rhee, S.C. et al. In Vivo evaluation of mixtures of uncultured freshly isolated adipose-derived stem cells and demineralized bone matrix for bone regeneration in a rat critically sized calvarial defect model. Stem Cells Dev 20, 233-242 (2011).
13. Buckland, M. et al. Aspirin-treated human DCs up-regulate ILT-3 and induce hyporesponsiveness and regulatory activity in responder T cells. Am J Transplant 6, 2046-2059 (2006).
14. Yamaza, T. et al. Pharmacologic stem cell based intervention as a new approach to osteoporosis treatment in rodents. PLoS ONE 3, e2615 (2008).
15. Kwon, M.S. et al. Effect of aspirin and acetaminophen on proinflammatory cytokine-induced pain behavior in mice. Pharmacology 74, 152-156 (2005).
16. Krebsbach, P.H. et al. Bone formation in vivo: comparison of osteogenesis by transplanted mouse and human marrow stromal fibroblasts. Transplantation 63, 1059-1069 (1997).
17. Batouli, S. et al. Comparison of stem cell-mediated osteogenesis and dentinogenesis. J Den Res 82, 975-980 (2003).

## Claims

1. A complex composition for tissue engineering or a tissue regeneration device, comprising:
at least one bone marrow mesenchymal stem cell;
an effective amount of aspirin;
a substrate; and
at least one CD4⁺CD25⁺Foxp3⁺ regulatory T cell.

2. The composition or device of claim 1, wherein said effective amount is about 50 µg/ml or more.

3. The composition or device of claim 1, wherein said substrate is a water-insoluble, non-elastic, porous, pliable material capable of absorbing or adsorbing the BMMSC and aspirin.

4. The complex composition according to any one of claims 1 to 3 for use in a method for promoting localized tissue regeneration, comprising: applying said tissue engineering complex composition to a site in need of tissue regeneration.

5. The complex composition for use according to claim 4, further comprising the step of pre-treating a plurality of bone marrow mesenchymal stem cells with aspirin before applying the complex to the site.

6. A method for forming the complex composition or the tissue regeneration device according to any one of claims 1 to 3, comprising: adding a predetermined amount of bone marrow mesenchymal stem cell, CD4⁺CD25⁺Foxp3⁺ regulatory T cells and aspirin to a substrate or a carrier.

7. A combination of CD4⁺CD25⁺Foxp3⁺ regulatory T cells and aspirin for use in a method of promoting tissue regeneration at a bone marrow mesenchymal stem cell (BMMSC) implantation site of a subject, comprising: administering the CD4⁺CD25⁺Foxp3⁺ regulatory T cells to the subject systemically; and applying aspirin to the site.

## Patentansprüche

1. Eine Komplex-Zusammensetzung für Gewebezüchtung oder eine Geweberegenerations-Vorrichtung, umfassend:
mindestens eine mesenchymale Knochenmarkstammzelle;
eine wirksame Menge an Aspirin;
einen Träger; und
mindestens eine CD4⁺CD25⁺Foxp3⁺ regulatorische T-Zelle.

2. Die Zusammensetzung oder die Vorrichtung gemäß Anspruch 1, wobei die wirksame Menge etwa 50 µg/ml oder mehr beträgt.

3. Die Zusammensetzung oder die Vorrichtung gemäß Anspruch 1, wobei der Träger ein wasserunlösliches, nicht elastisches, poröses, formbares Material ist, dass zur Absoprtion fähig ist oder zur Adsorption des BMMSC und Aspirin fähig ist.

4. Die Komplex-Zusammensetzung gemäß einem der Ansprüche 1 bis 3 zur Verwendung in einem Verfahren zum Fördern von lokalisierter Geweberegenration, umfassend: das Aufbringen der Komplex-Zusammensetzung für Gewebezüchtung auf eine Stelle, die Geweberegeneration benötigt.

5. Die Komplex-Zusammensetzung zur Verwendung gemäß Anspruch 4, ferner umfassend den Schritt der Vorbehandlung einer Vielzahl an mesenchymalen Knochenmarkstammzellen mit Aspirin, bevor der Komplex auf die Stelle aufgebracht wird.

6. Ein Verfahren zum Bilden der Komplex-Zusammensetzung oder der Geweberegenerations-Vorrichtung gemäß einem der Ansprüche 1 bis 3, umfassend: das Zugeben einer vorgegebenen Menge von mesenchymalen Knochenmarkstammzellen, CD4⁺CD25⁺Foxp3⁺ regulatorische T-Zellen und Aspirin zu einem Substrat oder einem Träger.

7. Eine Kombination von CD4⁺CD25⁺Foxp3⁺ regulatorischen T-Zellen und Aspirin zur Verwendung in einem Verfahren zur Förderung der Geweberegenerierung an einer mesenchymalen Knochenmarksstammzellen (BMMSC) Implantatstelle eines Subjekts, umfassend: systemisches Verabreichen der CD4⁺CD25⁺Foxp3⁺ regulatorischen T-Zellen zu dem Subjekt; und Aufbringen des Aspirins auf die Stelle.

## Revendications

1. Composition de complexe pour le génie tissulaire ou un dispositif de régénération tissulaire, comprenant :
au moins une cellule souche mésenchymateuse de la moelle osseuse ;
une quantité efficace d'aspirine ;
un substrat ; et
au moins une cellule T régulatrice CD4⁺CD25⁺Foxp3⁺.

2. Composition ou dispositif selon la revendication 1, où ladite quantité efficace est environ 50 µg/ml ou plus.

3. Composition ou dispositif selon la revendication 1, où ledit substrat est un matériau insoluble dans l'eau, non élastique, poreux et souple capable d'absorber ou d'adsorber la CSMMO et l'aspirine.

4. Composition de complexe selon l'une quelconque des revendications 1 à 3 destinée à être utilisée dans un procédé pour favoriser une régénération tissulaire localisée, comprenant : l'application de ladite composition de complexe de génie tissulaire au niveau d'un site nécessitant une régénération tissulaire.

5. Composition de complexe destinée à être utilisée selon la revendication 4, comprenant en outre l'étape consistant à prétraiter une pluralité de cellules souches mésenchymateuses de la moelle osseuse avec de l'aspirine avant d'appliquer le complexe au site.

6. Procédé pour former la composition de complexe ou le dispositif de régénération tissulaire selon l'une quelconque des revendications 1 à 3, comprenant : l'ajout d'une quantité prédéterminée de cellules souches mésenchymateuses de la moelle osseuse, de cellules T régulatrices CD4⁺CD25⁺Foxp3⁺ et d'aspirine à un substrat ou un support.

7. Combinaison de cellules T régulatrices CD4⁺CD25⁺Foxp3⁺ et d'aspirine destinée à être utilisée dans un procédé pour favoriser une régénération tissulaire au niveau d'un site d'implantation de cellules souches mésenchymateuses de la moelle osseuse (CSMMO) d'un sujet, comprenant : l'administration des cellules T régulatrices CD4⁺CD25⁺Foxp3⁺ au sujet par voie systémique ; et l'application d'aspirine au site.
